# EUROPEAN PATENT SPECIFICATION

(11) **EP 2 667 844 B1**
(45) Date of publication and mention of the grant of the patent: **21.11.2018**
(21) Application number: 12707143.9
(22) Date of filing: 24.01.2012
(51) Int. Cl.: A61K 8/11, A61Q 19/00, A61K 9/51, A61K 38/13, A61K 38/23, A61K 38/28, A61K 8/85, A61K 39/395, A61K 9/50, A61K 9/00, A61K 9/14, A61K 31/573, A61K 31/575, A61K 31/57, C07C 323/57, A61K 9/16

(54) **NANOPARTICLES FOR DERMAL AND SYSTEMIC DELIVERY OF DRUGS**
NANOPARTIKEL FÜR DERMALE UND SYSTEMISCHE ABGABE VON ARZEIMITTELN
NANOPARTICULES POUR L'ADMINISTRATION DERMIQUE ET SYSTÉMIQUE DE MÉDICAMENTS

(30) Priority: 24.01.2011 US 201161435640 P; 24.01.2011 US 201161435674 P
(43) Date of publication of application: 04.12.2013
(73) Proprietor: Yissum Research Development Company of the Hebrew University of Jerusalem Ltd., 9139002 Jerusalem (IL)
(72) Inventor: BENITA, Simon, 64585 Tel Aviv (IL); NASSER, Taher, 16950 Tur'an Village (IL); KARRA, Nour, 68061 Jaffa Tel Aviv (IL); BADIHI, Amit, 97225 Jerusalem (IL)
(74) Representative: Vossius & Partner Patentanwälte Rechtsanwälte mbB
(86) International application number: PCT/IL2012/050020
(87) International publication number: WO 2012/101639

(56) References cited:
- CN-A- 101 926 775
- DE-A1-102007 062 113
- FR-A1- 2 827 767
- JP-A- 2006 321 763
- JP-A- 2008 024 658
- JP-A- 2010 150 151
- US-A1- 2002 130 430
- US-A1- 2003 194 443
- US-A1- 2006 233 883
- US-A1- 2010 247 668
- US-B1- 6 482 441
- US-B2- 7 807 188
- DATABASE WPI Week 200562 Thomson Scientific, London, GB; AN 2005-599909 XP002681904, & JP 2005 213170 A (HOSOKAWA FUNTAI GIJUTSU KENKYUSHO KK) 11 August 2005 (2005-08-11)

## Description

### FIELD OF THE INVENTION

The present invention relates, in most general terms, to polymer based nanoparticles as defined in claim 1 for the dermal or systemic delivery of therapeutic compounds.

### BACKGROUND OF THE INVENTION

Dermal therapy is still a challenge due to the inability to bypass the skin and deliver sufficient amounts of therapeutic compounds, either hydrophilic or lipophilic, to the deep skin layers. The penetration and permeation of poorly absorbed active ingredients can be improved by the addition of specific enhancers to the formulation, by the use of colloidal delivery systems, especially nanoparticles. The benefits of nanoparticles in such applications have been shown recently in several scientific fields, but little is known about the potential penetration of nanoparticles through the different skin layers. Nanoparticles may exert biological effects, simply by virtue of their dimension (100 nm or less).

Encapsulation using nanoparticulate systems is an increasingly implemented strategy in drug targeting and delivery. Such systems have been proposed for topical administration to enhance percutaneous transport into and across the skin barrier. However, the mechanism by which such particulate formulations facilitate skin transport remains ambiguous. These nanometric systems present a large surface area, a property that renders them very promising delivery systems for dermal and transdermal delivery. Their small particle size ensures close contact with the stratum corneum and the ability to control the particle diameter may modulate the skin site deep layer localization [1].

In a recent study, confocal laser scanning microscopy (CLSM) was used to visualize the distribution of non-biodegradable, fluorescent, polystyrene nanoparticles (diameters 20 and 200 nm) across porcine skin. The surface images revealed that (i) polystyrene nanoparticles accumulated preferentially in the follicular openings, (ii) this distribution increased in a time-dependant manner, and (iii) the follicular localization was favored by the smaller particle size. Apart from follicular uptake, localization of nanoparticles in skin "furrows" was apparent from the surface images. However, cross-sectional images revealed that these non-follicular structures did not offer an alternative penetration pathway for the polymer vectors, which transport was clearly impeded by the stratum corneum [2].

Recently, lipid nanoparticles have shown a great potential as vehicles for topical administration of active substances, principally owing to the possible targeting effect and controlled release in different skin strata. Ketoprofen and naproxen loaded lipid nanoparticles were prepared, using hot high pressure homogenization and ultra sonication techniques, and characterized by means of photocorrelation spectroscopy and differential scanning calorimetry. Nanoparticle behavior on human skin was assessed, *in vitro,* to determine drug percutaneous absorption (Franz cell method) and *in vivo* to establish the active localization (tape-stripping technique) and the controlled release abilities (UVB-induced erythema model). Results demonstrated that the particles were able to reduce drug penetration, increasing, simultaneously, the permeation and the accumulation in the horny layer. A prolonged anti-inflammatory effect was observed in the case of drug loaded nanoparticles with respect to the drug solution. Direct as well as indirect evidences corroborate the early reports on the usefulness of lipid nanoparticles as carriers for topical administration, stimulating new and deeper investigations in the field [3].

Polymeric nanocapsules have also been proposed as carriers for active agents for topical application. Among the many advantages of such delivery systems is the ability of the polymeric shell to achieve sustained release of the active ingredient and increase the sensitive compounds, thus resulting in an improved therapeutic effect of dermatological formulations. Currently, several commercially available cosmetic products have incorporated nanoparticles for the encapsulation of vitamin A, rose extract and wheat germ oil [**4**].

Another very recent paper published by Wu *et al.* [**5**] shows that polystyrene and poly(methyl methacrylate) nanoparticles were not able to pass beyond the most superficial layers of the skin, i.e., Stratum Corneum, following a 6 hours topical application; even polystyrene nanoparticles as small as 30 nm were not able to penetrate beyond the Stratum Corneum. On the other hand, the hydrophobic compound encapsulated inside the nanoparticles was released and was able to diffuse across the deeper layers of the skin.

The fact that nanoparticles are retarded at the skin surface may be an advantage, since the active ingredient can be slowly released over a prolonged period and diffuse across the skin barrier, while the nanoparticles themselves will not be systemically translocated. Thus, the authors [**5**] suggest that the penetration of nanoparticles across intact skin seems unlikely to induce a systemic effect.

Nevertheless, health authorities are very attentive to the potential negative effects that may be induced by non biodegradable nanoparticles within and across the skin following topical application. In fact, starting November 2009, member states of the EU have adopted a single regulation for cosmetic products: this was in fact the first national legislation to incorporate rules relating to the use of nanomaterials in any cosmetic products [**6**]. According to this regulation, anyone who wishes to distribute a new nanomaterials containing product will be required to hand out safety information to the European Commission prior to entry to the market. It should be stressed that these concerns are related to the use of non biodegradable nanoparticles, whereas, the use of nanoparticles that will be degraded in the skin over a reasonable period of time is not expected to elicit any adverse effect especially if the degradation products are safe.

In the 1970s, biodegradable polymers were suggested as appropriate drug delivery materials circumventing the requirement of polymer removal [**7**]. Aliphatic polyesters such as poly(ε-caprolactone) (PCL), poly(3-hydroxybutyrate) (PHB), poly(glycolic acid) (PGA), poly(lactic acid) (PLA) and its copolymers with glycolic acid i.e., poly(D,L-lactide-coglycolide) (PLGA) [**8-11**] have been widely used to formulate the controlled release devices. The reason why PLA and PLGA are widely used in the preparation of micro and nanoparticles, lies in the fact that they are nontoxic, well tolerated by the human body, biodegradable and biocompatible [**12-13**]. PLA and PGLA are FDA approved polymers for subcutaneous and intramuscular injections.

The degradation process of PLGA, also known as bulk erosion, occurs by autocatalytic cleavage of the ester bonds through spontaneous hydrolysis into oligomers and D,L-lactic and glycolic acid monomers [**14**]. Lactic acid enters the tricarboxylic acid cycle and is metabolized and eliminated as CO₂ and water. Glycolic acid is either excreted unchanged in the urine or enters the Krebs cycle and is also eliminated as CO₂ and water.

Recently the suitability of biodegradable poly-lactic acid nanoparticles (PLA, MW 30,000), loaded with fluorescent dyes as carriers for transepidermal drug delivery, was investigated in human skin explants using fluorescence microscopy, confocal laser scanning microscopy and flow cytometry [**15**]. The results showed that PLA particles penetrated into 50% of the vellus hair follicles, reaching a maximal depth corresponding to the entry of the sebaceous gland in 12-15% of all observed follicles. The accumulation of particles in the follicular ducts was accompanied by the release of dye to the viable epidermis and its retention in the sebaceous glands for up to 24h. Kinetic studies in vitro as well as in skin explants revealed destabilization of the particles and significant release of incorporated dye occurred upon contact with organic solvents and the skin surface. According to the authors these results suggest that particles based on PLA polymers may be ideal carriers for hair follicle and sebaceous gland targeting.

### REFERENCES

**[1]** Alves MP, Scarrone AL, Santos M, Pohlmann AR and Guterres SS (2007) Human skin penetration and distribution of nimesulide from hydrophilic gels containing nanocarriers. International journal of pharmaceutics 341(1-2):215-220.
**[2]** Alvarez-Roman R, Naik A, Kalia YN, Guy RH and Fessi H (2004) Skin penetration and distribution of polymeric nanoparticles. J Control Release 99(1):53-62.
**[3]** Puglia C, Blasi P, Rizza L, Schoubben A, Bonina F, Rossi C and Ricci M (2008) Lipid nanoparticles for prolonged topical delivery: an in vitro and in vivo investigation. International journal of pharmaceutics 357(1-2):295-304.
**[4]** Wu X, Price GJ and Guy RH (2009b) Disposition of nanoparticles and an associated lipophilic permeant following topical application to the skin. Molecular pharmaceutics 6(5): 1441-1448.
**[5]** Wu X, Griffin P, Price GJ and Guy RH (2009a) Preparation and in vitro evaluation of topical formulations based on polystyrene-poly-2-hydroxyl methacrylate nanoparticles. Molecular pharmaceutics 6(5): 1449-1456.
**[6]** Bowman DM, van Calster G and Friedrichs S (2010) Nanomaterials and regulation of cosmetics. Nature nanotechnology 5(2):92.
**[7]** Jalil R and Nixon JR (1990) Biodegradable poly(lactic acid) and poly(lactide-co-glycolide) microcapsules: problems associated with preparative techniques and release properties. Journal of microencapsulation 7(3):297-325.
**[8]** Vert M, Schwach G, Engel R and Coudane J (1998) Something new in the field of PLA/GA bioresorbable polymers? J Control Release 53(1-3):85-92.
**[9]** Jain RA (2000) The manufacturing techniques of various drug loaded biodegradable poly(lactide-co-glycolide) (PLGA) devices. Biomaterials 21(23):2475-2490.
**[10]** Uhrich KE, Cannizzaro SM, Langer RS and Shakesheff KM (1999) Polymeric Systems for Controlled Drug Release. Chemical Reviews 99(11):3181-3198.
**[11]** Park TG (1995) Degradation of poly(lactic-co-glycolic acid) microspheres: effect of copolymer composition. Biomaterials 16(15):1123-1130.
**[12]** Pistner H, Bendix DR, Muhling J and Reuther JF (1993) Poly(L-lactide): a long-term degradation study in vivo. Part III. Analytical characterization. Biomaterials 14(4):291-298.
**[13]** Mordenti J, Thomsen K, Licko V, Berleau L, Kahn JW, Cuthbertson RA, Duenas ET, Ryan AM, Schofield C, Berger TW, Meng YG and Cleland J (1999) Intraocular pharmacokinetics and safety of a humanized monoclonal antibody in rabbits after intravitreal administration of a solution or a PLGA microsphere formulation. Toxicol Sci 52(1):101-106.
**[14]** Li S (1999) Hydrolytic degradation characteristics of aliphatic polyesters derived from lactic and glycolic acids. Journal of biomedical materials research 48(3):342-353.
**[15]** Rancan F, Papakostas D, Hadam S, Hackbarth S, Delair T, Primard C, Verrier B, Sterry W, Blume-Peytavi U, Vogt A (2009) Investigation of polylactic acid (PLA) nanoparticles as drug delivery systems for local dermatotherapy. Pharm Res 26(8):2027-36.
**[16]** Mitragotri S (2004) Breaking the skin barrier. Advanced drug delivery reviews 56(5):555-556.
**[17]** Tan G, Xu P, Lawson LB, He J, Freytag LC, Clements JD and John VT (2010) Hydration effects on skin microstructure as probed by high-resolution cryo-scanning electron microscopy and mechanistic implications to enhanced transcutaneous delivery of biomacromolecules. Journal of pharmaceutical sciences 99(2):730-740.
**[18]** Fessi H, Puisieux F, Devissaguet JP, Ammoury N and Benita S (1989) Nanocapsule formation by interfacial polymer deposition following solvent displacement. International Journal of Pharmaceutics 55 R1-R4.
**[19]** PCT publication no. WO 2010/091187
**[20]** PCT publication no. WO 2004/084871
**[21]** US patent application no. US 2010/0247668
**[22]** PCT publication no. WO 2010/059253

JP2010150151 discloses compositions based on nanoparticles comprising PLGA and hyaluronic acid (= therapeutic active agent) and a carrier being adapted for transdermal application.

JP2006321763A discloses nanoparticles (nanocapsule; see abstract) based on PLGA-PEG polymers, the PLGA having a mw of 15000-25 000 Da, the particles size being of 300nm or less up to 50 nm.

JP2005213170A is directed to cosmetic compositions based on PLGA nanoparticles, PLGA having a mw of 15000-25000 Da, the particles size being from 50 to 250 nm (paragraphs 1, 9, 13, 39-40) which contain within the particles fat soluble vitamins.

US 7,807,188 discloses compositions based on PLGA nanoparticles with a mw of 13000 Da (comparative ex. 3, 4).

CN 101926775A discloses PLGA-PEG nanoparticles with a size of about 290 nm, the mw of PLGA being 8000-500000 da.

JP2008024658A discloses nanoparticles based on PLGA with a mw of 20000 (PLGA 7520) and having a size of 254nm.

US 2002/130430A1 discloses in examples 1, 3, 4 nanoparticles based on PLGA with a mw of 3000 (5050d12a / table 2), a particle below 500 nm (table 4, entry ms12; table 6, entires ms21-22, 24-25) containing insulin or cytochrome-C.

### SUMMARY OF THE INVENTION

The present invention is based on a novel approach for the construction of therapeutic vehicles, which by themselves or in combination with various active therapeutic agents have the ability to penetrate the skin and induce a therapeutic effect. Where the vehicles are associated with active agents, they are capable of delivering sufficient amounts of the agents, either hydrophilic or lipophilic, to the deep skin layers, to thereby induce either a topical or a systemic effect. While the invention may be utilized primarily to deliver therapeutic agents via the skin or other tissue barriers, it may also be utilized to deliver therapeutic agents via numerous other routes of administration, e.g., oral, i.v., i.m, s.c ophthalmic, etc. as further disclosed herein. The vehicles of the invention are able to cross biological membranes, provide the ability to simultaneously deliver more than one agent to a desired site, in particular both hydrophobic and hydrophilic agents, and most importantly, are able to deliver macromolecules which administration is otherwise impeded or not possible. As may be appreciated, known nanoparticulate delivery systems such as liposomes and nano-emulsions are limited in their ability, mainly because such systems cannot incorporate significant concentrations of hydrophilic macromolecules and/or enhance their penetration and prolonged residence time in the upper layers of the skin.

The nanoparticle vehicles of the invention possess a long physicochemical shelf-life over long storage periods, as freeze-dried powders, which can maintain their initial properties upon reconstitution with the addition of purified or sterile water prior to use.

The invention disclosed herein is based on a nanoparticle which may be used *per se* (i.e. without additional active agents where the therapeutic effect is denoted by the particle itself), or may be modified to carry one or more therapeutic agents. The nanoparticle of the invention is able, naked or comprising additional therapeutic agents, to penetrate into a tissue barrier, e.g., skin, to at least the 10 superficial epidermis layers, to a depth of at least 4-20 µm (micrometers). The nanoparticles biodegrade in the skin layer into which they penetrate and can thus, in addition to the effect that may be exerted by the associated therapeutic agent, mainly a hydrating or moisturizing effect, provide xerosis cutis treatment (dry skin) to the penetrable tissue by lactic acid and glycolic acid or only the lactic acid for a period of at least 24 hours, 72 hours, and even for a period of weeks.

The scope of protection of the invention is as defined in the claims.

Thus, in a first aspect of the invention there is provided a poly(lactic glycolic) acid (PLGA) nanoparticle having an average diameter of at most 500 nm, the PLGA having an average molecular weight of between 2,000 and 20,000 Da, wherein said nanoparticle being associated with at least one agent selected from a hydrophilic therapeutic agent conjugated to or associated with the surface of said nanoparticle, and a lipophilic therapeutic agent contained within said nanoparticle

In some embodiments, the invention provides a poly(lactic glycolic) acid (PLGA) nanoparticle having an average diameter of at most 500 nm, the PLGA having an average molecular weight of between 2,000 and 20,000 Da, the nanoparticle being surface-associated to at least one hydrophilic therapeutic agent.

In further embodiments, the invention provides a PLGA nanoparticle having an average diameter of at most 500 nm, the PLGA having an average molecular weight of between 2,000 and 20,000 Da, the nanoparticle containing therein at least one lipophilic therapeutic agent; namely, the nanoparticle may entrap or encapsulate the lipophilic therapeutic agent.

As a person of skill in the art would understand, the therapeutic agent may be associated with the surface of the nanoparticle or may be contained within said nanoparticle as further explained below. In some embodiments, the therapeutic agent is not contained within the nanoparticles, namely the core or matrix of the nanoparticles is essentially free of such therapeutic agents.

In some embodiments, the PLGA has an average molecular weight of between 2,000 and 10,000 Da. In other embodiments, the PLGA has an average molecular weight of between 2,000 and 7,000 Da. In other embodiments, the PLGA has an average molecular weight of between 2,000 and 5,000 Da. In still further embodiments, the PLGA has an average molecular weight of between 4,000 and 20,000 Da, or between 4,000 and 10,000 Da, or between 4,000 and 5,000 Da. In still other embodiments, the PLGA has an average molecular weight of about 2,000, about 4,500, about 5,000, about 7,000, or about 10,000 Da.

As used herein, the "***nanoparticle***" of the invention is a particulate carrier, a nanocapsule (NC) or a nanosphere (NS), which is biocompatible and sufficiently resistant to chemical and/or physical destruction, such that a sufficient amount of the nanoparticles remain substantially intact after administration into the human or animal body and for sufficient time to be able to reach the desired target tissue (organ). Generally, the nanoparticles are spherical in shape, having an average diameter of up to 500 nm. Where the shape of the particle is not spherical, the diameter refers to the longest dimension of the particle.

In some embodiments, the average diameter is between about 10 and 50 nm. In further embodiments, the average diameter is at least about 50 nm.

In some embodiments, the average diameter is between about 100 and 200 nm. In other embodiments, the average diameter is between about 200 and 300 nm. In further embodiments, the average diameter is between about 300 and 400 nm. In further embodiments, the average diameter is between about 400 and 500 nm.

In other embodiments, the average diameter is between about 50 and 500 nm. In other embodiments, the average diameter is between about 50 and 400 nm. In further embodiments, the average diameter is between about 50 and 300 nm. In further embodiments, the average diameter is between about 50 and 200 nm. In further embodiments, the average diameter is between about 50 and 100 nm. In further embodiments, the average diameter is between about 50 and 75 nm. In further embodiments, the average diameter is between about 50 and 60 nm.

The nanoparticles may each be substantially of the same shape and/or size. In some embodiments, the nanoparticles have a distribution of diameters such that no more than 0.01 percent to 10 percent of the particles have a diameter greater than 10 percent above or below the average diameter noted above, and in some embodiments, such that no more than 0.1, 0.2, 0.4, 0.6, 0.8, 1, 2, 3, 4, 5, 6, 7, 8, or 9 percent of the nanoparticles have a diameter greater than 10 percent above or below the average diameters noted above.

The PLGA polymer is a copolymer of polylactic acid (PLA) and polyglycolic acid (PGA), the copolymer being, in some embodiments, selected amongst block copolymer, random copolymer and grafted copolymer. In some embodiments, the copolymer is a random copolymer.

In some embodiments, the nanoparticles are of PLGA listed as Generally Recognized as Safe (GRAS) under Sections 201(s) and 409 of the Federal Food, Drug, and Cosmetic Act, and are approved for use in microparticulate systems.

In some embodiments, the average molecular weight of each of PLA and PGA, independently of the other, as present in the copolymer, is between 2,000 and 20,000 Da. In some embodiments, the PLA monomer is present in the PLGA in excess amounts. In other embodiments, the molar ratio of PLA to PGA is selected amongst 95:5, 90:10, 85:15, 80:20, 75:25, 70:30, 65:35, 60:40, 55:45 and 50:50. In some embodiments, the PLA to PGA molar ratio is 50:50 (1:1).

In some embodiments, the nanoparticle is formed of a random copolymer of equimolar PLA and PGA, wherein the copolymer has a molecular weight of at least 4,500 Da, and is in the form of a nanoparticle having an average diameter between 100 and 200 nm.

Nanoparticles of the invention or those utilized in accordance with the invention, which by themselves have a therapeutic effect (without an additional active agent) are used mainly for moisturizing/hydration purposes in cases of excess skin dryness that accompanies medical conditions, such as: atopic and contact dermatitis, psoriasis, eczema, thyroid disorders, ichtyosis, scleroderma, Sjorgen's disease and others.

The nanoparticles according to the invention may be used as such to induce at least one effect, e.g., therapeutic effect, or may be associated with at least one agent, e.g., therapeutic agent, which is capable of inducing, enhancing, arresting or diminishing at least one effect, by way of treatment or prevention of unwanted conditions or diseases in a subject. The at least one agent (substance, molecule, element, compound, entity, or a combination thereof) may be selected amongst therapeutic agents, i.e., agents capable of inducing or modulating a therapeutic effect when administered in a therapeutically effective amount, and non-therapeutic agents, i.e., which by themselves do not induce or modulate a therapeutic effect but which endow the nanoparticles with a selected characteristic, as will be further disclosed hereinbelow.

The at least one therapeutic agent may be selected amongst vitamins, proteins, anti-oxidants, peptides, polypeptides, lipids, carbohydrates, hormones, antibodies, monoclonal antibodies, vaccines and other prophylactic agents, diagnostic agents, contrasting agents, nucleic acids, nutraceutical agents, small molecules (of a molecular weight of less than about 1,000 Da or less than about 500 Da), electrolytes, drugs, immunological agents and any combination of any of the aforementioned.

In some embodiments, the at least one agent is a macromolecule (molecular weight above 1000 Da), which delivery through the skin layers is otherwise not possible. Such macromolecules may be lipophilic.

In some embodiments, the at least one therapeutic agent is selected from calcitonin, cyclosporin, insulin, dexamethasone, dexamethasone palmitate, cortisone, prednisone and others.

For certain applications, the at least one therapeutic agent is selected in accordance with its molecular weight. Thus, the at least one therapeutic agent may be selected to have a molecular weight higher than 1,000 Da. In other embodiments, the agent is selected to have a molecular weight of no more than 300 Da. In further embodiments, the agent is selected to have a molecular weight of between 500 and 1,000 Da.

In some embodiments, the nanoparticles of the invention may be further associated with a non-active agent. The non-active agent (non-therapeutic agnet) may be selected to modulate at least one characteristic of the nanoparticle, such characteristic may for example be one or more of size, polarity, hydrophobicity/hydrophilicity, electrical charge, reactivity, chemical stability, clearance rate, distribution, targeting and others.

In some embodiments, the non-active agent is a substantially linear carbon chain having at least 5 carbon atoms, and may or may not have one or more heteroatoms in the linear carbon chain.

In some embodiments, the non-active agent is selected from polyethylene glycols (PEG) of varying chain lengths, fatty acids, amino acids, aliphatic or non-aliphatic molecules, aliphatic thiols, aliphatic amines, and others. The agents may or may not be charged.

In some embodiments, the non-active agent is a fatty amino acid (alkyl amino acid). In the invention as claimed, the alkyl portion of said alkyl amino acid has between 10 and 30 carbon atoms and may be linear or branched, saturated, semi saturated or unsaturated. In further embodiments, the amino acid portion of said alkyl amino acid may be selected amongst natural or non-natural amino acids, and/or amongst alpha- and/or beta-amino acids.

In some embodiments, the nanoparticle may be non-PEGylated, i.e. the non-active agent is different from PEG.

Thus, depending on various parameters (which may be therapeutic or non-therapeutic) associated with the at least one agent, e.g., therapeutic or non-active, (the parameters being, for example, solubility, molecular weight, polarity, hydrophobicity/hydrophilicity, electrical charge, reactivity, chemical stability, biological activity, and others), the agent may be contained (encapsulated) in said nanoparticle, embedded in the polymer matrix making up the nanoparticle and/or chemically or physically associated with the surface (whole surface or a portion thereof) of the nanoparticle. For the chosen application, the nanoparticle may therefore be in the form of core/shell (termed hereinafter also as nanocapsule), having a polymeric shell and a core which may be empty of an active material or contain at least one agent.

Alternatively the nanoparticles are of a substantially uniform composition not featuring a distinct core/shell structure. These nanoparticles are herein referred to as nanospheres (NSs). In some embodiments, the inner part (core or inner matrix) of the nanoparticles are devoid of the at least one hydrophilic agent but can contain lipophilic agent dispersed or dissolved in the core or matrix, namely, the at least one hydrophilic agent may reside on or be associated with the surface of the nanoparticles.

In other embodiments, the nanoparticles consist essentially of PLGA.

Where nanocapsules (NCs) are employed, the at least one (active) lipophilic agent may be contained within the nanoparticles core (cavity), e.g., in an oily matrix, surrounded by a shell of the PLGA copolymer.

In some embodiments, at least one therapeutic agent is associated with the surface of the nanoparticle and at least one different therapeutic agent is associated to be contained within a core of said nanoparticle or within a matrix of said nanoparticle.

In some embodiments, the nanoparticles are nanocapsules containing at least one hydrophobic agent (the agent being contained in an oil core and thus is lipophilic). Depending on a particular intended application, the oily core may be selected amongst any oily organic solvent or medium (single material or mixture), such materials may be selected, in a non-limiting fashion, from octanoic acid, oleic acid, glyceryl tributyrate, long chain triglycerides (such as soybean) and others.

Alternatively, relatively uniform structures, e.g., nanospheres may be employed, where the at least one agent may be embedded within the nanoparticles matrix, e.g., homogenously, resulting in a nanoparticle in which the concentration of the active agent within the nanoparticle is uniform.

In some embodiments, modification of the nanoparticles (either nanocapusles or nanospheres) surface may be required to enhance the effectiveness of the nanoparticles in the delivery of a therapeutic agent. For example, the surface charge of the nanoparticles may be modified to achieve modified biodegradation and clearance of the nanoparticles. The porosity of the polymer element of the particle (whether the core in the nanocapsule or the uniform matrix in the nanosphere) may also be optimized to achieve extended and controlled release of the therapeutic agent.

In another manifestation of the invention, the nanoparticles are modified to permit association therewith with at least one (therapeutic or non-therapeutic, or targeting) agent; the association may be a chemical association, such as covalent bonding, electrostatic bonding, ionic interaction, dipole-dipole interaction, hydrophilic interaction,van der Waal's interaction, hydrogen bonding, or a physical association of at least a portion of the agent with the nanoparticle. The physical association may be such that at least a portion of the at least one agent (or a linker moiety associated therewith) is entrapped, embedded, adsorbed or anchored into the nanoparticle element or surface. Herein, the physical association is referred to in general as "***physical anchoring***".

A nanoparticle may be associated with one or more of a variety of agents, either therapeutic or non-therapeutic. For example, when two or more agents are used, they can be similar or different. Utilization of a plurality of agents in a particular nanoparticle can allow the targeting of multiple biological targets or can increase the affinity for a particular target. In addition, the nanoparticle may contain two agents, each having a different solubility- one hydrophobic (e.g., in the core) and one hydrophilic (e.g., in the shell or extending out of the particle).

The association between each of the nanoparticles and the various agents may be selected, based on the intended application, to be labile, namely undergo dissociation under specific conditions, or non-labile. Typically, where the at least one agent is a therapeutic agent, it is either associated with the surface of the nanoparticles via labile bond(s) or via one or more linker moieties.

In some embodiments, the at least one agent is a therapeutic agent which association with the nanoparticles is via one or more linker moieties, the linker moiety being bifunctional, namely having a first (e.g., hydrophobic) portion which is capable of association (interaction) with the surface of the nanoparticles, and a second (e.g., hydrophilic) portion which is capable of association with the therapeutic agent.

The nanoparticle associated with a plurality of such linker moieties is referred to herein as a "***modified nanoparticle***", namely a nanoparticle, as defined, which at least a part of its surface is associated with linker moieties which are capable of undergoing association with at least one agent. The plurality of linkers interacting with the surface of the nanoparticles, need not all be associated with therapeutic agents. Some may be associated with other non-therapeutic agents; others may have bare end-groups (unassociated with any agent). In some embodiments, the linkers are associated with one or more different therapeutic agents.

The association between the linker and the nanoparticle surface is typically selected from covalent bonding, electrostatic bonding, hydrogen bonding and physical anchoring (non-covalent) of at least a portion of the linker into the nanoparticle surface. The association between the linker and the at least one therapeutic agent is selected from covalent bonding, electrostatic bonding, and hydrogen bonding.

In some embodiments, the linker moiety is associated with one or both of (a) the at least one therapeutic agent and (b) the nanoparticle surface via covalent bonding. In other embodiments, the association between the linker and the nanoparticle surface is via anchoring (e.g., in the surface of the nanoparticle and may penetrate into the solid/oil core of the nanoparticle) of at least a portion of the linker into the nanoparticle surface, with another portion of the linker exposed and extending away from the nanoparticle surface.

In further embodiments, the linker is covalently bonded to said at least one therapeutic agent. In some embodiments, one or both of (a) the association of the linker with the therapeutic agent and (b) the association with the linker with the nanoparticle surface is labile.

In some embodiments, in the nanoparticle having anchored (non-covalently) on its surface a plurality of linker moieties, each of said plurality of linker moieties is covalently bonded to at least one agent; both surface anchoring and covalent boding are labile.

The association of the linker and any of the nanoparticles and the agent may be labile, namely the linker may be a readily cleavable linker, which is susceptible to dissociation under conditions found *in vivo.* For example, where the nanoparticles of the invention are employed as drug delivery systems for skin applications, topical or systemic, upon passing into and through one or more skin layers, the therapeutic may be released from the linker or the nanoparticles carrier. Readily cleavable associations can be such that are cleaved by an enzyme of a specific activity or by hydrolysis. For skin applications, the association between the linker and the therapeutic or between the nanoparticles and the linker may be selected to be cleavable by an enzyme present in one or more layers of skin tissue.

In some embodiments, the linker moiety contains a carboxylic acid group (to form esters) or a thiol group (to form a sulfide bond).

In other embodiments, the linker moiety is selected according to the half-life of the cleavable association, namely the quantity of the therapeutic that becomes dissociated from the linker. In some embodiments, the association of the linker to the therapeutic has a half-life of between 1 minute and 48 hours. In some embodiments, the half-life is less than 24 hours.

In further embodiments, the linker moiety comprises a functional group selected from -S-, -NH-, -C(=O)O-, -C(=O)S-, -C(=O)NH-, -C(=S)NH-, -OC(=O)NH-, -NH(=O)NH-, -S(=O)NH-, -S(=O)₂NH-, and others.

In some embodiments, the linker is selected amongst polyethylene glycols (PEG) of varying chain lengths. PEG linkers may also be employed in combination with other linkers for the purpose of eluding the immune system and fending off attacking degradative enzymes.

In some embodiments, the linker moiety is a fatty amino acid (alkyl amino acids), wherein the alkyl portion has between 10 and 30 carbon atoms and may be linear or branched, saturated, semi saturated or unsaturated. The amino acid portion may be selected amongst natural or non-natural amino acids, and/or amongst alpha- and/or beta-amino acids. The amino acid group of the linker may be derivable an amino acid selected, without limitation, from alpha and beta amino acids. In the invention as claimed, the linker is a fatty cystein having an alkyl chain of at least 10 carbon atoms.

In further embodiments, the linker is oleylcysteineamide of the formula I:

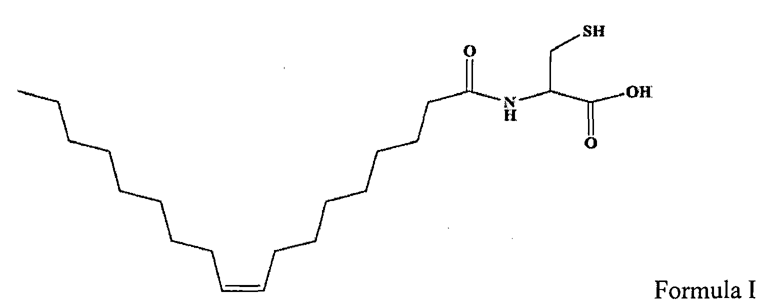

In some embodiments, the linker moiety is a thiolated compound, and thus the modified nanoparticle is a thiolated nanoparticle capable of association with, e.g., macromolecules (molecular weight above 1000 Dalton), hydrophilic molecules and electrolytes. The association between the thiolated nanoparticle and the agent may be via an active group on the agent, such group may be a maleimide functional group.

The present invention also provides a polymeric nanoparticle having on its surface a plurality of therapeutic agents, each agent being associated (bonded) to said nanoparticle via a linker moiety, the nanoparticles being of a polymeric material selected from poly(lactic acid) (PLA), poly(lacto-co-glycolide) (PLG), poly(lactic glycolic) acid (PLGA), poly(lactide), polyglycolic acid (PGA), poly(caprolactone poly(hydroxybutyrate) and/or copolymers thereof. In some embodiments, said polymeric material is selected from PLA, PGA and PLGA. In further embodiments, the polymeric nanoparticles are of PLGA.

In some embodiments, the linker moiety is oleylcysteineamide. In other embodiments, the nanoparticle has the physical characteristics disclosed hereinabove. In some embodiments, the nanoparticle is a poly(lactic glycolic) acid (PLGA) nanoparticle having an average diameter of at most 500 nm, the PLGA having an average molecular weight of up to 20,000 Da.

The nanoparticles of the invention may be used in the preparation of pharmaceutical compositions for medical use. In some embodiments, the compositions are used in methods of therapeutic treatments, namely- treatment and/or prevention of skin disorders, diseases of the eye, and any other disease which may be treatable by the compositions of the invention.

The concentration of nanoparticles in a pharmaceutical composition may be selected so that the amount is sufficient to deliver a desired effective amount of a therapeutic agent to the subject, and in accordance with the particular mode of administration selected. As known, the "***effective amount***" for purposes herein may be determined by such considerations as known in the art. The amount must be effective to achieve the desired therapeutic effect, depending, *inter alia,* on the type and severity of the disease to be treated and the treatment regime. The effective amount is typically determined in appropriately designed clinical trials (dose range studies) and the person versed in the art will know how to properly conduct such trials in order to determine the effective amount. As generally known, the effective amount depends on a variety of factors including the affinity of the ligand to the receptor, its distribution profile within the body, a variety of pharmacological parameters such as half life in the body, on undesired side effects, if any, on factors such as age and gender, and others.

The pharmaceutical composition of the invention may comprise varying nanoparticle types or sizes, of different or same dispersion properties, utilizing different or same dispersing materials.

The nanoparticles may also be used as drug or bioactive delivery systems to transport a wide range of therapeutic agents topically, orally, by inhalation, nasally, or parenterally into the circulatory system following administration. The nanoparticle delivery systems of the invention facilitate targeted drug delivery and controlled release applications, enhance drug bioavailability at the site of action also due to a decrease of clearance, reduce dosing frequency, and minimize side effects.

In most general terms, the delivery system of the invention comprises:
(i) a polymeric nanoparticle as disclosed herein; and
(ii) at least one agent associated with said nanoparticle, said at least one agent being optionally associated with said surface via a linker moiety.

In some embodiments, the linker has a first portion physically anchored (non-covalently associated) to said surface and a second portion associated with said at least one agent. In some embodiments, the first portion physically anchored to said surface is hydrophobic, and the second portion associated with said at least one agent is hydrophilic.

The delivery system of the invention is capable of delivering the therapeutic agent at a rate allowing controlled release of the agent over at least about 12 hours, or in some embodiments, at least about 24 hours, or in other embodiments, over a period of 10-20 days. As such, the delivery system may be used for a variety of applications, such as, without limitation, drug delivery, gene therapy, medical diagnosis, and for medical therapeutics for, e.g., skin pathologies, cancer, pathogen-borne diseases, hormone-related diseases, reaction-by-products associated with organ transplants, and other abnormal cell or tissue growth.

The delivery systems are typically administered as pharmaceutical compositions, comprising the system and a pharmaceutically acceptable carrier. The pharmaceutically acceptable carrier may be selected from vehicles, adjuvants, excipients, and diluents, which are readily available to the public. The pharmaceutically acceptable carrier is selected to be chemically inert to the delivery system of the invention or to any component thereof and one which has no detrimental side effects or toxicity under the conditions of use.

The invention provides compositions formulated for a variety of applications. In some embodiments are provided compositions adapted for transdermal administration, e.g., for delivery of a therapeutic into the circulatory system of a subject. In further embodiments are provided compositions for topical administration. The topical composition is typically employed for delivering a therapeutic agent across the Stratum corneum. In further embodiments are provided compositions adapted for oral administration of a therapeutic agent. Further provided are compositions adapted for ophthalmic administration of a therapeutic agent. The ophthalmic compositions may be administered as eye drops or via injection into the eye.

In some embodiments, an ophthalmic composition is provided which comprises at least one nanoparticle according to the invention, said nanoparticle being associated with a therapeutic macromolecule, the association being optionally via at least one linker. In some embodiments, the composition is in a form suitable for interoccular injection or in the form of eye drops.

The choice of carrier will be determined in part by the particular therapeutic agent, as well as by the particular method used to administer the composition or the delivery system. Accordingly, the pharmaceutical composition or the delivery system of the present invention may be formulated for oral, enteral, buccal, nasal, topical, transepithelial, rectal, vaginal, aerosol, transmucosal, epidermal, transdermal, dermal, ophthalmic, pulmonary, subcutaneous, intradermal and/or parenteral administration routes. In some embodiments, the pharmaceutical composition or delivery system is administered transdermally, topically, subcutaneously and/or parenterally.

The delivery system can be administered in a biocompatible aqueous or lipid solution. This solution can be comprised of, but not limited to, saline, water or a pharmaceutically acceptable organic medium.

In some embodiments, the composition of the invention is essentially free of water.

The administration of delivery system formulation can be carried out at a single dose or at a dose repeated once or several times after a certain time interval. The appropriate dosage may vary according to such parameters as the therapeutically effective dosage as dictated by and directly dependent on the individual being treated, the mode of administration, the unique characteristics of the therapeutic agent and the particular therapeutic effect to be achieved. Appropriate doses can be established by the person skilled in the art.

The pharmaceutical composition of the present invention may be selected to treat, prevent or diagnose any pathology or condition. The term "***treatment***" or any lingual variation thereof, as used herein, refers to the administering of a therapeutic amount of the composition or system of the present invention which is effective to ameliorate undesired symptoms associated with a disease, to prevent the manifestation of such symptoms before they occur, to slow down the progression of the disease, slow down the deterioration of symptoms, to enhance the onset of remission period, slow down the irreversible damage caused in the progressive chronic stage of the disease, to delay the onset of said progressive stage, to lessen the severity or cure the disease, to improve survival rate or induce more rapid recovery, or to prevent the disease from occurring or a combination of two or more of the above

Pharmaceutical compositions of the present invention may be particularly advantageous to those tissues protected by physical barriers. Such barriers may be the skin, a blood barrier (e.g., blood-thymus, blood-brain, blood-air, blood-testis, etc), organ external membrane and others. Where the barrier is the skin, the skin pathologies which may be treated by the pharmaceutical compositions of the invention include, but are not limited to antifungal disorders or diseases, acne, psoriasis, vitiligo, a keloid, a burn, a scar, xerosis, ichthoyosis, keratosis, keratoderma, dermatitis, pruritis, eczema, skin cancer, and a callus.

The pharmaceutical compositions of the invention may be used to prevent or treat any dermatologic condition. In some embodiments, the dermatological condition is selected amongst dermatologic diseases, such as dermatitis, eczema, contact dermatitis, allergic contact dermatitis, irritant contact dermatitis, atopic dermatitis, infantile eczema, Besnier's prurigo, allergic dermatitis, flexural eczema, disseminated neurodermatitis, seborrheic (or seborrhoeic) dermatitis, infantile seborrheic dermatitis, adult seborreic dermatitis, psoriasis, neurodermatitis, scabies, systemic dermatitis, dermatitis herpetiformis, perioral dermatitis, discoid eczema, Nummular dermatitis, Housewives' eczema, Pompholyx dyshidrosis, Recalcitrant pustular eruptions of the palms and soles, Barber's or pustular psoriasis, Generalized Exfoliative Dermatitis, Stasis Dermatitis, varicose eczema, Dyshidrotic eczema, Lichen Simplex Chronicus (Localized Scratch Dermatitis; Neurodermatitis), Lichen Planus, Fungal infection, Candida intertrigo, tinea capitis, white spot, panau, ringworm, athlete's foot, moniliasis, candidiasis; dermatophyte infection, vesicular dermatitis, chronic dermatitis, spongiotic dermatitis, dermatitis venata, Vidal's lichen, asteatosis eczema dermatitis, autosensitization eczema, or a combination thereof.

In further embodiments, the compositions of the invention may be used to prevent or treat pimples, acne vulgaris, birthmarks, freckles, tattoos, scars, burns, sun burns, wrinkles, frown lines, crow's feet, café-au-lait spots, benign skin tumors, which in one embodiment, is Seborrhoeic keratosis, Dermatosis papulosa nigra, Skin Tags, Sebaceous hyperplasia, Syringomas, Xanthelasma, or a combination thereof; benign skin growths, viral warts, diaper candidiasis, folliculitis, furuncles, boils, carbuncles, fungal infections of the skin, guttate hypomelanosis, hair loss, impetigo, melasma, molluscum contagiosum, rosacea, scapies, shingles, erysipelas, erythrasma, herpes zoster, varicella-zoster virus, chicken pox, skin cancers (such as squamos cell carcinoma, basal cell carcinoma, malignant melanoma), premalignant growths (such as congenital moles, actinic keratosis), urticaria, hives, vitiligo, Ichthyosis, Acanthosis Nigricans, Bullous Pemphigoid, Corns and Calluses, Dandruff, Dry Skin, Erythema Nodosum, Graves' Dermopathy, Henoch-Schönlein Purpura,, Keratosis Pilaris:, Lichen Nitidus, Lichen Planus, Lichen Sclerosus, Mastocytosis, Molluscum Contagiosum, Pityriasis Rosea, Pityriasis Rubra Pilaris, PLEVA, or Mucha-Habermann Disease, Epidermolysis Bullosa, Seborrheic Keratoses, Stevens-Johnson Syndrome, Pemphigus, or a combination thereof.

In further embodiments, the compositions of the invention may be used to prevent or treat insect bites or stings.

In additional embodiments, the compositions of the present invention may be used to prevent or treat dermatologic conditions that are associated with the eye area, such as Syringoma, Xanthelasma, Impetigo, atopic dermatitis, contact dermatitis, or a combination thereof; the scalp, fingernails, such as infection by bacteria, fungi, yeast and virus, Paronychia, or psoriasis; mouth area, such as Oral Lichen Planus, Cold Sores (Herpetic Gingivostomatitis), Oral Leukoplakia, Oral Candidiasis, or a combination thereof; or a combination thereof.

As known, human skin is made of numerous layers which may be divided into three main group layers: Stratum corneum which is located on the outer surface of the skin, the epidermis and the dermis. While the Stratum corneum is a keratin-filled layer of cells in an extracellular lipid-rich matrix, which in fact is the main barrier to drug delivery into skin, the epidermis and the dermis layers are viable tissues. The epidermis is free from blood vessels, but the dermis contains capillary loops that can channel therapeutics for transepithelial systemic distribution.

While transdermal delivery of drugs seems to be the route of choice, only a limited number of drugs can be administered through this route. The inability to transdermally deliver a greater variety of drugs depends mostly on the requirement for low molecular weight (drugs of molecular weights not higher than 500 Da), lipophilicity and small doses of the drug. The delivery system of the invention clearly overcomes these obstacles. As noted above, the system of the invention is able of holding therapeutic agents of a great variety of molecular weights and hydrophilicities. The delivery system of the invention permits the transport of the at least one therapeutic agent across at least one of the skin layers, across the Stratum corneum, the epidermis and the dermis layers. Without wishing to be bound by theory, the ability of the delivery system to transport the therapeutic across the Stratum corneum depends on a series of events that include diffusion of the intact system or the dissociated therapeutic agent and/or the dissociated nanoparticles through a hydrated keratin layer and into the deeper skin layers.

Thus, the invention also provides a delivery system comprising:
(i) a PLGA nanoparticle as defined herein; and
(ii) at least one therapeutic agent associated with said nanoparticle, said at least one therapeutic agent being optionally associated with said surface via a linker moiety having, or is alternatively contained within said nanoparticle.

Further provided is a multistage delivery system which comprises:
(i) a polymeric nanoparticle as disclosed herein;
(ii) a linker moiety associated with the surface of said polymeric nanoparticles;
(iii) at least one therapeutic agent associated with said linker moiety; and
(iv) optionally at least one additional agent which may be associated with the nanoparticle.

With the ability of the delivery system of the invention to dissociate under biological conditions, the multistage system provides one or more of the following advantages: (1) the multistage system permits the transport of the therapeutic agent through a tissue barrier by various mechanisms; (2) the therapeutic agent may be dissociated from the linker or from the nanoparticle in cases where the agent is directly associated with the nanoparticle and thus deliverable to a particular target tissue or organ in the body of a subject administered with the delivery system; and (3) the modified nanoparticle, comprising the polymeric nanoparticle and the linker moiety (free of the therapeutic agent) may further travel through additional barrier tissues, increasing their hydration and inducing additional therapeutic effects; and (4) where the nanoparticles are nanocapsules also holding an agent within the capsule core, they may allow for simultaneous delivery and localization of a plurality of therapeutic agents.

Accordingly, in the delivery system of the invention, each component may be designed to have a separate intended function, which may be different from an intended function of another component. For example, the therapeutic agent may be designed to target a specific site, which may be different from a site targeted by the linker moiety or the bare nanoparticle, and thus overcome or bypass a specific biological barrier, which may be different from the biological barrier being overcome or bypassed the system as a whole. For example, where the at least one agent is an antibody linked to the nanoparticle, it can bind to specific antigens on the surface of the cells in the epidermis or dermis while the agent within the core of the nanoparticle can be released earlier by simple diffusion. Furthermore, the incorporated agent can be mostly released from the nanoparticles while the nanoparticle can be fragmented or biodegraded more slowly and be eliminated through the dermis as monomers of PLA or PGA.

The invention also provides a process, as defined in the set of claims, for the preparation of a delivery system according to the invention, the process comprising:
- obtaining a nanoparticle, as defined herein;
- reacting said nanoparticle with a linker moiety under conditions permitting association between the nanoparticle surface and the linker moiety, to thereby obtain a surface-modified nanoparticle; and
- contacting the surface modified nanoparticle with at least one agent, e.g., therapeutic or non-therapeutic, to allow association between the linker end group; to thereby obtain a delivery system in accordance with the present invention.

In some embodiments, the linker moiety may be associated with the therapeutic agent prior to the contacting with the nanoparticle and the process may thus comprise:
- obtaining a nanoparticle, as define herein;
- obtaining a therapeutic agent associated linker moiety; and
- reacting the therapeutic agent associated linker with said nanoparticle to permit association of at least a portion of said linker with the surface of the nanoparticle.

In some embodiments, the delivery system/multistage system comprises nanoparticles associated with oleylcysteineamide, which is anchored at the interface of nanoparticles and thus may be easily applied to various PLA and PLGA polymer mixtures of different molecular weights, thereby resulting in a wide range of thiolated nanoparticles.

The linking process does not require *a priori* chemical modification of the particle-forming polymer. This is achieved by the use of a molecular linker, e.g., oleylcysteineamide, having a lipophilic portion which non-covalently anchors to the particle's polymeric matrix or polymeric nanocapsule wall and a second portion comprising a thiol compound to which it is possible, in a subsequent step, to bind the desired therapeutic agent either directly or activated by a maleimide group. This approach eliminates the need to tailor for each different therapeutic agent a different nanoparticle composition, and enables a generic linker (with an active therapeutic), which can be used for different therapeutic applications.

Other than employing the methods available for chemically associating the therapeutic agent to the linker, e.g., carbodimide mediated conjugation, the thiol modified nanoparticle surface may be used also or alternatively for the chelation and dermal delivery of vital electrolytes, e.g., divalent metals, such as copper, selenium, calcium, magnesium and zinc. The thiolated nanoparticles may also serve as a delivery system to chelate undesired excess amounts of metals and thus reduce the metal catalyzed ROS (Reactive Oxygen Species) mediated deleterious effect on the skin.

The invention also provides a poly(lactic glycolic) acid (PLGA) nanoparticle, the PLGA having an average molecular weight of between 2,000 and 20,000 Da, said nanoparticle being surface-associated to at least one agent (therapeutic or non-therapeutic), and having an average diameter of at most 500 nm, the nanoparticles being obtainable by a process comprising:
- obtaining a PLGA nanoparticle having an average diameter of at most 500 nm, the PLGA having an average molecular weight of between 2,000 and 20,000 Da;
- reacting said nanoparticle with a linker moiety under conditions permitting association between the nanoparticle surface and the linker moiety, to thereby obtain a surface-modified nanoparticle; and
- contacting the surface-modified nanoparticle with at least one agent being selected from a therapeutic or non-active agent, to allow association between the linker end group with said at least one agent. The PLGA nanoparticles obtained by said process are as defined in the set of claims.

Also provided is a process, as defined in the set of claims, for the preparation of a poly(lactic glycolic) acid (PLGA) nanoparticle, the PLGA having an average molecular weight of between 2,000 and 20,000 Da, said nanoparticle being surface-associated to at least one agent, and having an average diameter of at most 500 nm, the process comprising:
- obtaining a PLGA nanoparticle having an average diameter of at most 500 nm, the PLGA having an average molecular weight of between 2,000 and 20,000 Da;
- reacting said nanoparticle with a linker moiety under conditions permitting association between the nanoparticle surface and the linker moiety, to thereby obtain a surface-modified nanoparticle; and
- contacting the surface-modified nanoparticle with at least one agent being selected from a therapeutic or non-active agent, to allow association between the linker end group with said at least one agent.

In some embodiments, in the processes and products produced thereby, the linker moiety (e.g., oleylcysteineamide) is associated with the at least one agent prior to the association with the nanoparticle. The at least one agent is typically a therapeutic agent.

Further provided is a process for the preparation of a poly(lactic glycolic) acid (PLGA) nanoparticle, the PLGA having an average molecular weight of between 2,000 and 20,000 Da, said nanoparticle being surface-associated to at least one agent, and having an average diameter of at most 500 nm, the process comprising:
- obtaining a PLGA nanoparticle having an average diameter of at most 500 nm, the PLGA having an average molecular weight of between 2,000 and 20,000 Da;
- reacting said nanoparticle with at least one agent being selected from a therapeutic or non-therapeutic agent, to allow association between the at least one agent and the surface of said nanoparticle.

Also provided are polylactic acid (PLA) nanoparticles having an average diameter of at most 500 nm, the PLA having an average molecular weight of up to 10,000Da.

In some embodiments, the PLA has an average molecular weight of between 1,000 and 10,000 Da. In other embodiments, the PLA has an average molecular weight of between 1,000 and 5,000 Da. In further embodiments, the PLA has an average molecular weight of between 1,000 and 3,000 Da. In still other embodiments, the PLA has an average molecular weight of about 1,000, about 2,000, about 3,000, about 4,000 or about 5,000 Da.

Further provided are uses of oleylcysteineamide in processes for preparing delivery systems for delivering therapeutic agents to a subject, said processes comprising reacting said oleylcysteineamide to a therapeutic agent to be delivered to said subject.

Also provided is oleylcysteineamide for use in association with at least one nanoparticle.

Further provided is a macromolecule chemically associated (e.g., via covalent bonding) to oleylcysteineamide.

Also provided is a PLGA nanoparticle having on its surface a plurality of surface-exposed thiol groups, said thiol groups being activated for association with at least one agent selected from a therapeutic agent and a non-therapeutic agent, as disclosed herein. In some embodiments, said thiol groups are of oleylcysteineamide.

### BRIEF DESCRIPTION OF THE DRAWINGS

In order to understand the invention and to see how it may be carried out in practice, embodiments will now be described, by way of non-limiting example only, with reference to the accompanying drawings, in which:
**Figs. 1A-B** are CRYO-TEM images of blank PLGA₄₅₀₀ nanoparticles at various areas of the carbon grid (**Fig. 1A**) and blank PLGA₄₅₀₀ nanoparticles at various areas of the carbon grid following one month storage at 4°C (**Fig. 1B**).
**Figs. 2A-B** are CRYO-TEM images of DHEA loaded PLGA₄₅₀₀ nanocapsules at various areas of the carbon grid (**Fig. 2A**) and DHEA loaded PLGA₅₀₀₀₀ nanocapsules at various areas of the carbon grid (**Fig. 2B**).
**Fig. 3** is a collection of fluorescent images of various consecutive tape-stripping following topical administration over 3h of different NIR-PLGA nanosphere formulations (2.25mg/cm²). Scanning was performed using ODYSSEY® Infra Red Imaging System.
**Figs. 4A-D** is a depiction of reconstructed fluorescent images of whole skin specimens, 2h following topical administration of DiD incorporated nanocapsules or nanospheres (4.5mg/cm²). **Fig. 4A****-** DiD loaded PLGA₄₅₀₀ nanospheress; **Fig. 4B-**DiD loaded PLGA₅₀₀₀₀ nanospheres; **Fig. 4C****-** DiD control solution; **Fig. 4D****-** DiD loaded PLGA₄₅₀₀ nanocapsules. Z stack scanning was performed using a Zeiss LSM 710 confocal microscope.

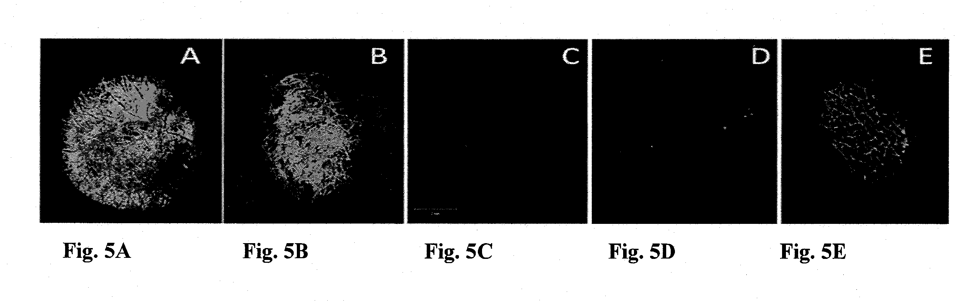

**Figs. 5A-E** is a depiction of reconstructed fluorescent images of whole skin specimens, 2h following topical administration of varied fluorescent nanocapsules or nanospheres (3.75mg/cm²). **Fig. 5A****-** DiD incorporated and rhodamine B conjugated PLGA₄₅₀₀ nanospheres; **Fig. 5B****-** DiD incorporated and rhodamine B conjugated PLGA₄₅₀₀ nanocapsules; **Fig. 5C****-** Rhodamin B incorporated latex nanospheres; **Fig. 5D****-** DiD and rhodamine B conjugated PLGA₄₅₀₀ aqueous dispersion control; **Fig. 5E****-** DiD and rhodamine B conjugated PLGA₄₅₀₀ MCT containing aqueous dispersion control. Z stack scanning was performed using a Zeiss LSM 710 confocal microscope.
**Figs. 6A-B** exhibits DiD (**Fig. 6A**) and Rhodamine B (**Fig. 6B**) cumulative fluorescence intensity as a function of skin depth following 2 hours topical administration of various DiD incorporated RhdB-PLGA formulations (3.75mg/cm2) using 27 µm incremental optical sectioning.
**Figs. 7A-D** CLSM images of 8 µm thick vertical skin sections 2h after topical administration of DID incorporated RhdB-PLGA NPs (**Fig. 7A**) and NCs (**Fig. 7B**) and their respective controls (**Fig. 7C** and **Fig. 7D**) (3.75 mg/cm²). Bar =100 µm.
**Fig. 8** exhibits Rhodamine B cumulative fluorescence intensity as a function of skin depth following 2 hours topical administration of various rhodamine B incorporated formulations including PLGA nanospheres, nanocapsules and latex nanspheres (3.75mg/cm²) using 27 µm incremental optical sectioning.

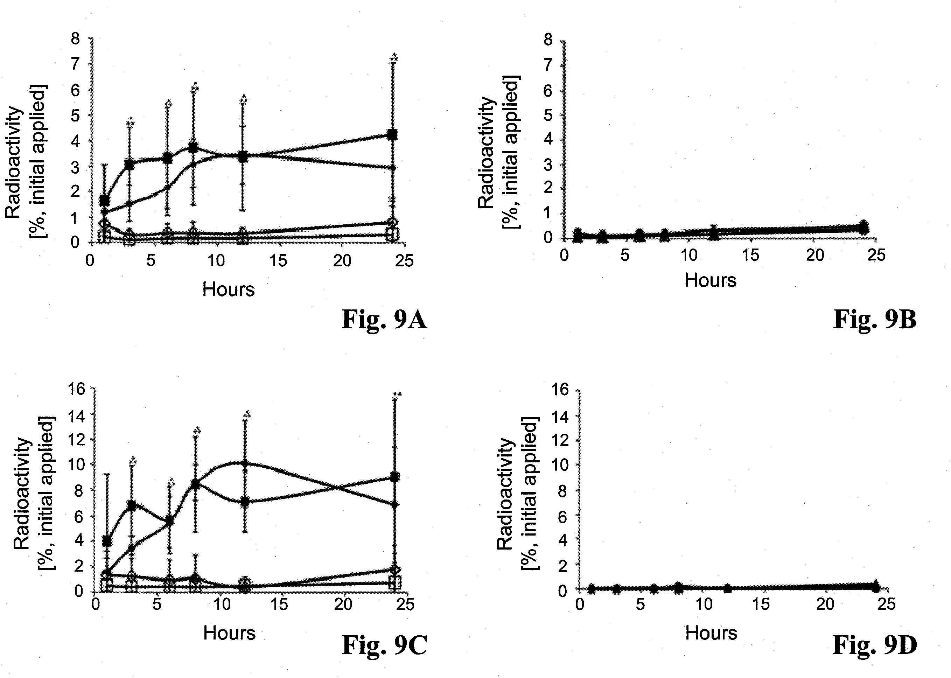

**Figs. 9A-D** [³H]DHEA (**Fig. 9A** and **Fig. 9C**) and [³H]COE (**Fig. 9B** and **Fig. 9D**) distribution in the viable epidermis (**Fig. 9A** and **Fig. 9B**) and dermis (**Fig. 9C** and **Fig. 9D**) skin compartments over time following incubation of various radioactive nanocarriers and their respective controls. **Fig. 9A** and **Fig. 9C**: positively (◆) and negatively (■) charged [³H]DHEA NCs and their respective oil controls (◊,□); **Fig. 9B** and **Fig. 9D**: [³H]COE NSs (▲), [³H]COE NCs (●) and their respective controls (Δ,○). Significant difference (*P value* <0.05) of the positively (*) and negatively (**) charged DHEA NCs in comparison to their respective controls
**Fig. 10** exhibits [³H]DHEA amounts recorded in the receptor compartment fluids following topical application of positive (◆) and negative (■) DHEA loaded NCs and their respective oily controls (◊,□). Values are mean ±SD. Significant difference (*P value* <0.05) of the positively (*) and negatively (**) charged DHEA NCs in comparison to their respective controls.
**Figs. 11A-C** are transmission electron microscopy microphotography of cetuximab immunonanoparticles (INPs) following incubation over 1h, using goat anti-human IgG secondary antibody conjugated to 12nm gold particle at different magnifications.
**Figs. 12A-C** are flow cytometry histograms demonstrating the binding of cetuximab immune nanoparticles to A549 cells. Depicted are surface activated nanoparticles (**Fig. 12A**) and rituximab (isotype matched) immunonanoparticles (**Fig. 12B**) at increasing concentrations (0.025/µg/ml, 0.05/µg/ml, 0.1µg/ml, 05µg/ml and 1µg/ml) (**Fig. 12C**). 0.1µg/ml, 0.5µg/ml and 1/µg/ml equivalents of cetuximab INPs compared to 1µg/ml equivalent of rituximab immune nanoparticles (full background histogram).
**Figs. 13A-E** are reconstructed fluorescent images of whole skin specimens, 3h following topical administration of various immunological and reference nanoparticulate formulations (6mg/cm² eq. to 0.12 mg MAb/cm²), following specific immunohistochemistry staining. Scanning was performed using an Olympus confocal microscope.
**Fig. 14** depicts individual fluorescence intensities per cm² calculated separately in up to twelve consecutive ∼35 µm sections, following topical administration of various immunological and reference nanoparticulate formulations (6mg/cm² eq. to 0.12 mg MAb/cm²), and specific immunohistochemistry staining.
**Fig. 15** depicts extrapolated cumulative fluorescent intensities per cm² calculated for up to 385 µm, following topical administration of various immunological and reference nanoparticulate formulations (6mg/cm² eq. to 0.12 mg MAb/cm²), and specific immunohistochemistry staining.
**Fig. 16** depicts calculated AUC values of cumulative fluorescent intensities per cm² calculated for up to 385 µm, following topical administration of various immunological and reference nanoparticulate formulations (6mg/cm² eq. to 0.12 mg MAb/cm²), and specific immunohistochemistry staining.

### DETAILED DESCRIPTION OF THE INVENTION

### I. Lactic acid and glycolic delivery to the skin

Use is made of the clinically well-accepted PLGA polymers as well as PLA particles of a specific molecular weight, to prepare nanoparticles of a certain particle size that are applied onto the skin, penetrate in the upper layers of the dermis and release, in a controlled manner over time, lactic and glycolic acid, or only lactic acid, which are natural moisturizing factors, allowing a prolonged and sustained hydration of the skin without being harmful.

The PLGA nanoparticles, *per se*, empty or loaded with appropriate actives are used as the prolonged active hydrating ingredients, as a result of their degradation within the skin leading to the progressive and continuous release of lactic and glycolic acid. Even if the nanoparticles penetrate into the deep layer of the epidermis or even the dermis, they do not induce any damage as previously described since the hydrolysis product lactic and glycolic acids are naturally eliminated or excreted.

It should be emphasized the PLGA (or PLA), as the active hydrating components of the composition of the invention, are not merely used as carriers for delivery of other components to the skin, although the invention also encompasses the possibility that other beneficial active components are used. Thus, in accordance with the invention the composition is intended for topical application, i.e., contains carriers for topical applications, as well as for other applications.

The nanoparticles of the invention are typically of a size smaller than 500nm. Typically, the nanoparticles are of a size range of between 100 and 200 nm, or between 50 and 100nm.

In some embodiments, the molecular weight of PLGA and the ratio between PLA and PGA is tailored so that the nanoparticles have the following properties:
(**a**) Penetrate into the skin to at least the 10 superficial epidermis layers;
(**b**) Penetrate to a depth of at least 4-20 micrometers into the skin;
(**c**) Biodegrade in the skin layer into which they penetrate (typically about 15% in the Stratum corneum);
(**d**) Sustained release of the lactic acid and glycolic acid or only the lactic acid for a period above 24 hours, preferably above 72 hours, more preferably about a week.

Without wishing to be bound by theory, there seems to be interplay between the size of particle (which influences the penetration rate and the deepness of penetration), the ratio of PLA and PGA and the molecular weight of the PLGA, in such a way that the above properties can be achieved by a number of combinations. Several changes in parameters may neutralize each other.

In some embodiments, the ratio of PLA:PGA is 85:15; 72:25; or 50:50. In some embodiments, the ratio is 50:50.

In other embodiments, the molecular weight of the PLGA ranges from 2,000 to 10,000 Da. In some embodiments, the ratio is between 2,000 and 4,000.

In other embodiments, the PLA particles may be employed *per se*, in such embodiments the PLA molecular weight is in the range of 4,000 and 20,000.

### II. Encapsulation strategies of insoluble compounds in nanoparticles-the potential of DHEA loaded PLGA nanoparticles

In the present invention, the nanoparticles may be loaded with active materials such as vitamins, peptides, and others as disclosed hereinabove.

Humans have adrenals that secrete large amounts of dehydroepiandrosterone (DHEA) and its sulphate derivatives (DHEAS). A remarkable feature of plasma DHEA(S) levels in humans is their great decrease with aging. Researchers have postulated that this age-related decline in DHEA(S) levels may explain some of the degenerative changes associated with aging. Three mechanisms of action of DHEA(S) have been identified. DHEA and DHEA(S) are precursors of testosterone and estradiol. DHEA(S) is a neurosteroid, which modulates neuronal excitability via specific interactions with neurotransmitter receptors, and DHEA is an activator of calcium-gated potassium channels.

Randomized, placebo-controlled clinical trials which included 280 healthy individuals (140 men and 140 women) aged 60-years and over treated with (near) physiological doses of DHEA (50 mg/day) over one year have yielded very positive results. Impact of DHEA replacement treatment was assessed on mood, well being, cognitive and sexual functions, bone mass, body composition, vascular risk factors, immune functions and skin. Interestingly, an improvement of the skin status was observed, particularly in women, in terms of hydration, epidermal thickness, sebum production, and skin pigmentation. Furthermore, no harmful consequences were observed following this 50mg/day DHEA administration over one year.

It is known that DHEA might be related to the process of skin aging through the regulation and degradation of extracellular matrix protein. It was demonstrated that DHEA can increase procollagen synthesis and inhibit collagen degradation by decreasing matrix metalloproteinase (MMP)-1 synthesis and increasing tissue inhibitor of matrix metalloprotease (TIMP-1) production in cultured dermal fibroblasts. DHEA (5%) in ethanol:olive oil (1:2) was topically applied to buttock skin of volunteers 12 times over 4 weeks, and was found to significantly increase the expression of procollagen alpha1 (I) mRNA and protein in both aged and young skin. On the other hand, topical DHEA significantly decreased the basal expression of MMP-1 mRNA and protein, but increased the expression of TIMP-1 protein in aged skin. These recent results suggest the possibility of using DHEA as an anti-skin aging agent.

Based on the overall reported results, exogenous DHEA, administered topically may promote keratinization of the epidermis, enhance skin hydration by increasing the endogenous production and secretion of sebum subsequently reinforcing the barrier effect of the skin, treat the atrophy of the dermis by inhibiting the loss of collagen and connective tissue and finally can modulate the pigmentation of the skin. These properties render DHEA the active of choice as an anti-aging active ingredient provided DHEA is adequately dissolved in the topical formulation, can diffuse from the formulation towards the skin and be fully bioavailable for skin penetration following dermal application. Indeed, DHEA exhibits complex solubility limitations in common cosmetic and pharmaceutical solvents such as water, polar oils and vegetable oils. DHEA is practically insoluble in water (0.02mg/ml) and is known for its tendency to precipitate rapidly within topical regular formulations even at concentrations lower than 0.5%, yielding several polymorphic crystal forms which are difficult to control and exhibit very slow dissolution rate. Furthermore, DHEA shows low solubility in lipophilic phases with a maximum solubility of 1.77% in mid chain triglycerides. The most accepted topical dosage form is the o/w emulsion in which the DHEA should be dissolved in the lipophilic phase. However, this solution is very difficult to accomplish since very high concentrations of oil phase (more than 70%) are needed to achieve a DHEA concentration eliciting an adequate efficacy activity (approximately 0.5% w/v). Topical products with such high oil phase concentrations will be unpleasant and unappealing, ruling out their usefulness as cosmetic products.

There is no doubt that the recrystallization process of DHEA should be prevented since it can potentially cause significant variations in therapeutic bioavailability and efficacy. The drug crystals need first to re-dissolve in the skin prior to diffusing and penetrating the superficial skin layers. Such a process is unlikely to occur easily and will significantly affect the activity of the product. Moreover, the recrystallization process can affect the stability and the physical appearance of the formulation. Thus, there is clearly a need to prepare pleasant and convenient o/w topical formulations where DHEA loaded nanoparticles can be dispersed at an adequate concentration precipitate out of the formulation. Furthermore, the DHEA embedded nanocarrier should be incorporated in a topical formulation, which can promote penetration of the active ingredient within the epidermis and dermis layers where its action is most needed.

### III. DELIVERY OF SURFACE BOUND MACROMOLECULES AND MINERALS INTO THE SKIN USING THIOL ACTIVATED NANOPARTICLES

Commercially available products utilizing transdermal delivery have been mainly limited to low molecular weight lipophilic drugs (MW<500 Da) [16], with larger molecular weights (MW>500 Da) facing penetration difficulties [17]. Due to the impervious nature of the Stratum corneum towards macromolecules, a suitable penetration enhancer should substantially improve transport of macromolecules through the skin. Various technologies have been developed for this purpose, including the use of microneedles, electroporation, laser generated pressure waves, hyperthermia, low-frequency sonophoresis, iontophoresis, penetration enhancers, or a combination of these methods. Many penetration enhancement techniques face inherent challenges, such as scale-up and safety concerns [17]. The present invention proposes the delivery of macromolecules, mostly hydrophilic, by a non invasive method, using a surface binding technique of macromolecules to thiolated nanoparticles.

### Thiolated NPs- State of the art

Nanoparticles were functionalized with a maleimide moiety, which were then conjugated to a thiolated protein. Alternatively, nanoparticles can be functionalized with a thiol group then conjugated to a maleimidic residue on the protein. Traditionally, such delivery systems have been mostly used for the targeted delivery of drug loaded nanoparticles, principally to malignant tumors, where the surface conjugated protein is used simply as a targeting moiety recognizing disease specific epitopes.

### IV. EXPERIMENTAL

### 1. DiD loaded PLGA NPs and NCs and/or Rhodamine B PLGA conjugated NPs or NCS preparation (reference example) :

PLGA was dissolved in acetone containing 0.2% w/v Tween 80, at a concentration of 0.6%w/v. In case were NCs were prepared, octanoic acid or MCT at a concentration of 0.13% w/v was also added to the organic phase. If DiD loaded NPs were prepared then, an aliquot of acetone DiD solution at a concentration of 1 mg/ml was also added to the organic phase, resulting in a final concentration of 15-30 µg/ml. If rhodamine B PLGA conjugated NPs or NCs were prepared, 0.03 %w/v rhodamine B tagged PLGA were dissolved in acetone together with 0.57 %w/v non labeled PLGA. The organic phase was added to the aqueous phase containing 0.1 % w/v Solutol® HS 15. The suspension was stirred at 900 rpm over 15 minutes and then concentrated by evaporation to a final polymer concentration of 30 mg/ml. The aqueous and oil control compositions were identical to the formulation described above, only without the polymer presence.

### 2. [³H]DHEA and [³H]COE PLGA solid nanoparticle encapsulation and evaluation

### DHEA NPs preparation (reference example)

DHEA loaded PLGA nanocapsules were prepared using the interfacial deposition method [**18**]. DHEA was solubilized in octanoic acid/MCT/oleic acid and in acetone. If positively charged DHEA NCs were prepared, the cationic lipid, DOTAP [1,2-dioleoyl-3-trimethylammonium-propane], at a concentration of 0.1% w/v was added to the organic phase. In case were radioactive DHEA NCs were prepared, 15 µCi of tritiated DHEA were inserted into the oil core of the NCs during the preparation of the NCs together with 1 mg of cold DHEA. In case *[³H]Cholesteryl oleyl ether* ([³H]COE) were prepared, 80 and 127 µCi [³H]COE were either dissolved in MCT to form NCs or simply added to the organic phase for NPs formation, respectively. The organic phase was added drop wise to the aqueous phase under stirring at 900 rpm, and the formulation was concentrated by evaporation to a polymer concentration of 8 mg/ml. The formulations were filtered through 0.8 µm membrane and then 3 ml from the different [³H]DHEA NCs were dia-filtrated with 30 ml PBS (pH 7.4) (Vivaspin 300,000 MWCO, Vivascience, Stonehouse, UK) and filtered through 1.2µm filter (w/0.8 µm Supor® Membrane, Pall corporation, Ann Arbor, USA). The radioactivity intensity for the overall formulations and their respective controls was set so a finite dose applied will be in the range of a total of 0.63-1.08 µCi/ml. The compositions of the organic phase and the aqueous phase are presented in **Table 1**.

**Table 1: compositions of organic phase and aqueous phase**

| **Organic phase** | **Aqueous phase** |
|---|---|
| PLGA 4500 MW - 150 mg | Solutol HS 15 - 50 mg |
| Octanoic acid - 75 µl | Water - 100 ml |
| DHEA - 10mg | |
| TWEEN 80 - 50 mg | |
| Acetone - 50 ml | |

**Particle size analysis:** mean diameter and particle size distribution measurements were carried out utilizing an ALV Noninvasive Back Scattering High Performance Particle Sizer (ALV-NIBS HPPS, Langen, Germany) at 25°C and using water as diluent.

**Zeta potential measurements**: the zeta potential of the NPs was measured using the Malvern zetasizer (Malvern, UK) diluted in HPLC grade water.

**Scanning** (**SEM**) **and Transmission electron microscopy** (**TEM**): morphological evaluation was performed by means of scanning and transmission TEM (Philips Technai F20 100 KV). Specimens for TEM visualization are prepared by mixing the sample with phosphotungstic acid 2% (w/v) pH 6.4 for negative staining.

### Cryo-Transmission electron microscopy (Cryo-TEM):

A drop of the aqueous phase was placed on a carbon-coated holey polymer film supported on a 300 mesh Cu grid (Ted Pella Ltd), the excess liquid was blotted and the specimen was vitrified via a fast quench in liquid ethane to -170°C. The procedure was performed automatically in the Vitrobot (FEI). The vitrified specimens were transferred into liquid nitrogen for storage. The fast cooling is known to preserve the structures present at the bulk solution and therefore provides direct information on the morphology and aggregation state of the objects in the bulk solution without drying. The samples were studied using a FEI Tecnai 12 G2 TEM, at 120kV with a Gatan cryo-holder maintained at -180°C, and images were recorded on a slow scan cooled charge-coupled device CCD Gatan manufactured camera. Images were recorded with the Digital Micrograph software package, at low dose conditions, to minimize electron beam radiation damage.

### 3. Diffusion experiments (reference example):

Franz diffusion cells (Crown Glass, Sommerville, NJ, USA) with an effective diffusion area of 1/0.2 cm² and an acceptor compartment of 8 ml were used. The receptor fluid was a phosphate buffer, pH 7.4.

Throughout the experiment, the receptor chamber content was continuously agitated by a small magnetic stirrer. The temperature of the skin was maintained at 32°C by water circulating system regulated at 37°C. Finite doses of the vehicle and formulations (10-50 mg polymer per cell) were applied on the horny layer of the skin or cellulose membrane. The donor chamber was opened to the atmosphere. The exact time of application was noted and considered as time zero for each cell. At 4, 8, 12 and 24h or 26h, the complete receptor fluid was collected and replaced with fresh temperature equilibrated receptor medium. The determination of the diffused active ingredient concentration was determined from aliquots. At the end of the 24- or 26-h period, the skin surface was washed 5 times with 100 ml of distilled water or ethanol. The washing fluids were pooled and an aliquot part (1 ml) was assayed for the active ingredient concentration.

The cells were then dismantled and the dermis separated from the epidermis by means of elevated temperature as described above. The active ingredient content was determined by means of HPLC or other validated analytical techniques. Furthermore, the presence of lactic or glycolic acid in the receptor medium was examined.

### 4. DiD loaded PLGA NPs and NCs and/or Rhodamine PLGA conjugated NPs or NCS site localization (reference example):

Excised human skin or porcine ear skin samples were placed on Franz diffusion cells (PermeGear, Inc., Hellertown, PA), with an orifice diameter of 5/11.28 mm, 5/8 mL receptor volume and an effective diffusion area of 0.2/1.0 cm². The receptor fluid was phosphate buffer, pH 7.4. Throughout the experiment, the receptor chamber content was continuously agitated by a small magnetic stirrer. The temperature of the skin was maintained at 32°C by water circulating system regulated at 37°C. The solutions and different NP and NCs formulations either loaded with entrapped DiD fluorescent probe with free PLGA or PLGA covalently bound to rhodamine B were applied on the skin as detailed below. This protocol was adopted to follow the skin localization of both the entrapped DiD probe and of the conjugated rhodamine B polymer. The various formulations were prepared as described in the experimental section above. The dose applied for each formulation on the excised skin samples was 125µl of a 30mg/ml PLGA polymer concentration with an initial entrapped fluorescent content of DiD 30µg/ml.

After single incubation period or at different time intervals, some of the skin samples were dissected to identify the localization site of the nanocarrier in the various skin layers by confocal microscope. The procedure was as follows using histological sectioning. The skin specimens were fixated using formaldehyde 4% for 30 minutes. The fixated tissues were placed in an adequate plastic cubic embedding in tissue freezing medium (OCT, Tissue-Tek). Skin samples were then deeply frozen at -80°C and vertically cut into 10 µm thick sections, utilizing Cryostat at -20°C. Then, the treated specimens were stored in a refrigerator untill to the confocal microscopic analysis.

In addition, some whole mount skin specimens were kept intact after Franz cells incubation at selected time interval of 2h and immediately observed by confocal microscope and further reconstructed using 3D imaging from z-stacks pictures. The fluorescence intensity versus skin depth for nanocarriers and respective controls using line profile (calculated intensity for each section and whole specimen accumulative intensity are reported). Samples data is provided in **Table 2.**

**Table 2: Description of the composition of each formulation topically applied with specific equivalent dose**

| **Formulation Composition** | **PLGA, mg/cm² (MW, kDa)** | **PLGA-rhodamine B conjugated % w/w from NPs** | **Oil core type in NCs (µl)** | **Volume applied (µl)** | **DiD eq. dose Applied (µg)** |
|---|---|---|---|---|---|
| DiD NPs | 4.5 (4) | - | - | 150 | 1.125 |
| DiD NPs | 4.5 (50) | - | - | 150 | 1.125 |
| DiD NCs | 4.5 (4) | - | Octanoic acid (75) | 150 | 1.125 |
| DiD micellar solution | - | - | - | 150 | 1.125 |
| DiD incorporated rhodamine B conjugated PLGA NPs | 3.75 (4) | 5 | - | 125 | 3.75 |
| DiD incorporated rhodamine B conjugated PLGA NCs | 3.75(4) | 5 | MCT (113) | 125 | 3.75 |
| Rhodamine B incorporated Latex NPs | 3.75(NA) | | - | 125 | - |
| DiD and rhodamine B conjugated PLGA aqueous dispersion | - | 5 | - | 125 | 3.75 |
| DiD and rhodamine B conjugated PLGA oil containing aqueous dispersion | - | 5 | MCT (113) | 125 | 3.75 |

### 5. [³H]DHEA NCs site localization and deep skin layer localization (reference example):

[³H]DHEA NCs formulations were applied on the skin using the Franz cell diffusion system. [³H]DHEA localization in the various skin layers was determined by skin compartment dissection technique. Dermatome pig skin (600-800 µm thick) was mounted on Franz diffusion cells (Crown Glass, Sommerville, NJ, USA) with an effective diffusion area of 1 cm² and an acceptor compartment of 8 ml (PBS, pH 7.4). At different time intervals, skin compartment dissection was carried out to identify the localization site of the nanocarriers in the skin surface, upper corneocytes layers, epidermis, dermis and receptor cell. First, the remainder of the formulation was collected following serial washings to allow adequate recovery. Then, the skin surface was removed by adequate sequential tapes stripping, contributing the first strip to the donor compartment. The rest of the viable epidermis was separated from the dermis by means of heat elevated temperature, and then chemically dissolved by solvable digestion liquid. Finally the receptor fluids were also collected and further analyzed.

In addition, in an attempt to reveal quantitatively the biofate of the NCs and NPs in the various layers of the skin, 80 and 127 µCi *[³H]Cholesteryl oleyl ether* ([³H]COE) were either dissolved in the oil core of the NCs or entrapped in the nanomatrices of the NPs respectively. The radioactive tracer, *[³H]Cholesteryl oleyl ether* ([³H]COE) is highly lipophilic with a log P above 15 (>15) and its localization within skin layers reflects the localization of either the oil core of the NC or the nanomatrix of the NP since the probe cannot be released from the nanocarriers in view of its extremely high lipophilicity.

### 6. Oleylcysteineamide synthesis and characterization Oleylcysteineamide synthesis

Under a flow of nitrogen the flask was charged via syringe with oleic acid (OA) (2.0g, 7.1 mmol), 60 ml of dry tetrahydrofuran, and triethylamine (0.5 ml, 7.1 mmol). Stirring was commenced, and the solution was cooled to an internal temperature of -15°C using a dry ice-isopropyl alcohol bath at -5° to -10°C. Ethyl chloroformate (0.87 ml, 6.1 mmol) was added and the solution was stirred for 5 min. The addition of ethyl chloroformate resulted in an internal temperature rise to +8 to +10°C and the precipitation of a white solid. Following the precipitation the continuously stirred mixture, still in the dry-isopropyl alcohol bath, was allowed to reach an internal temperature of -14°C. Cysteine (1.0 g, 8.26 mmol) dissolved in 5% Na₂CO₃ solution (10 ml) introduced into the flask via a syringe needle, was vigorously bubbled through the solution for 10 min with manual stirring: the internal temperature rises abruptly to 25°C. With the flask still in the cooling bath, stirring was continued for an additional 30 min, and the reaction mixture was stored in the freezer at -15°C overnight. The slurry was stirred with tetrahydrofuran (100 ml) at room temperature for 5 min and ammonium salts were removed by suction filtration through a Buchner funnel. After the solids were rinsed with tetrahydrofuran (20 ml), the filtrate was passed through a plug of silica gel (25 g Merck 60 230-400 mesh) in a coarse porosity sintered-glass filter funnel with aspirator suction. The funnel was further washed with acetonitrile (100 ml) and the combined filtrates were evaporated (rotary evaporator) to give a viscous liquid.

Formation of oleylcysteineamide was confirmed by H-NMR (Mercury VX 300, Varian, Inc., CA, USA) and LC-MS (Finnigan LCQDuo, ThermoQuest, NY, USA).

### Oleylcysteineamide Characterization

¹H-NMR (CDCl₃, δ): 0.818, 0.848, 0.868, 0.871, 0.889, 1.247, 1.255, 1.297, 1.391, 1.423, 1.452, 1.621, 1.642, 1.968, 1.989, 2.008, 2.174, 2.177, 2.268, 2.2932.320, 2.348, 3.005, 3.054, 4.881, 5.316, 5.325, 5.335, 5.343, 5.353, 5.369, 6.516, 6.540, 7.259 ppm.
LC-MS: Peak at: 384.42.

The NMR analysis confirms the formation of the linker oleylcysteineamide, while the LC-MS spectrum clearly corroborates the molecular weight of the product which is 385.6 g/mol

### 7. Preparation and characterization of surface activated nanoparticles and macromolecules conjugation:

Nanoparticles were prepared using the well established interfacial deposition method [**18**]. The oleylcysteineamide linker molecule was dissolved in the organic phase containing the polymer dissolved in water soluble organic solvent. The organic phase was then added drop wise to the aqueous phase which contains a surfactant. The suspension was evaporated at 37°C under reduced pressure to a final nanoparticulate suspension volume of 10 ml. A maleimide bearing spacer molecule (LC-SMCC) was reacted with the desired macromolecule at pH 8 for subsequent conjugation to the thiol moiety. The thiol activated NPs and the relevant maleimide bearing molecule were then mixed and allowed to react overnight under a nitrogen atmosphere. The following day, free unbound molecules were separated from the conjugated NPs using a dia-filtration method.

**Table 3: Formulation composition**

| **Organic phase** | **Aqueous phase** |
|---|---|
| Polymer 300mg | Solutol HS 15 100 mg |
| Oleyl cysteine 20 mg | Water 100 ml |
| Tween 80 100 mg | |
| Acetone 50 ml | |

### Size and zeta potential characterization:

The size and zeta potential of the various NPs were measured in water using a DTS zetasizer (Malvern, UK).

### Determination of the conjugation efficiency of the various macromolecules to NPs:

The conjugation efficiency of the macromolecules such as MAbs was determined using the calorimetric Bicinchoninic acid assay (BCA) for protein quantification (Pierce, IL, USA).

It should be noted, that the same procedure disclosed herein has been used to link hyaluronic acid to the nanoparticles.

### 8. Incorporation of nanoparticles into anhydrous cream

The advantages of dispersing the final product in anhydrous cream are enormous. Increasing amounts (0.1-10%) of freeze-dried powders of the NPs and the NPs prepared are incorporated into a novel cream comprising no water. The relative amounts of the ingredients of this cream are detailed in **Table 4**.

**Table 4: relative amounts of ingredients**

| **Ingredient** | **Relative amont/100** |
|---|---|
| Dow corning 9040 - Cyclopentasiloxane (and) Dimethicone crosspolymer | 40.0-50.0 |
| Dimethicone | 5.0-7.0 |
| Cyclopentasiloxane | 10.0-15.0 |
| Shin etsu KSG-16 Dimethicone (and) Dimethicone/Vinyl dimethicone Crosspolymer | 20.0-35.0 |
| Boron Nitride | 0.3 -0.70 |
| lauroyl Lysine Ajinomoto | 0.2 -0.70 |
| hyaluronic acid MP 50000 | 0.1-0.40 |
| Palmitoyloligopeptide - Biopeptide CL Sederma | 0.05 -0.3 |
| Palmitoyl tetrapeptide - N-Palmitoyl-Rigin | 0.05 -0.3 |

### IV. Preliminary results

### Nanoparticle formulation and characterization

Fluorescent nanoparticles were prepared to facilitate visual detection of the nanoparticles. PLA was conjugated to the fluorescent Rhodamine B probe. The nanoparticles were then prepared as described in the experimental section above.

The results demonstrate a homogenous nanoparticle formulation. It was possible to see the nanoparticles owing to the fluorescence labeling with Rhodamine fluorophore at excitation/emission 560/580 nm. The nanoparticles exhibited a mean diameter of 52 nm and a Zeta potential value of -37.3 mV.

This technique was used to detect and identify the localization of the nanoparticles with time in the various layers of the skin following topical application.

### Cryo-TEM visualization of PLGA biodegradable NPs one month following preparation

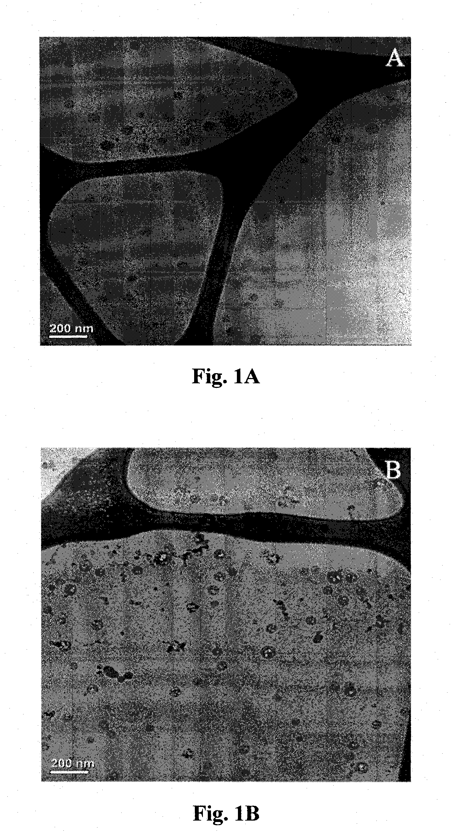

The Cryo-TEM images of blank PLGA₄₅₀₀ nanoparticles at various areas of the carbon grid are depicted in **Fig. 1A**. Nanoparticles appear quite homogenous in size and shape. Furthermore, cryo-TEM images of blank PLGA₄₅₀₀ nanoparticles at various areas of the carbon grid following one month storage at 4°C are depicted in **Fig. 1B****.** Nanoparticles are at different degradation stages. It can be noted that nanoparticles degraded with time in an aqueous environment.

### DHEA loaded PLGA nanoparticles (reference example)

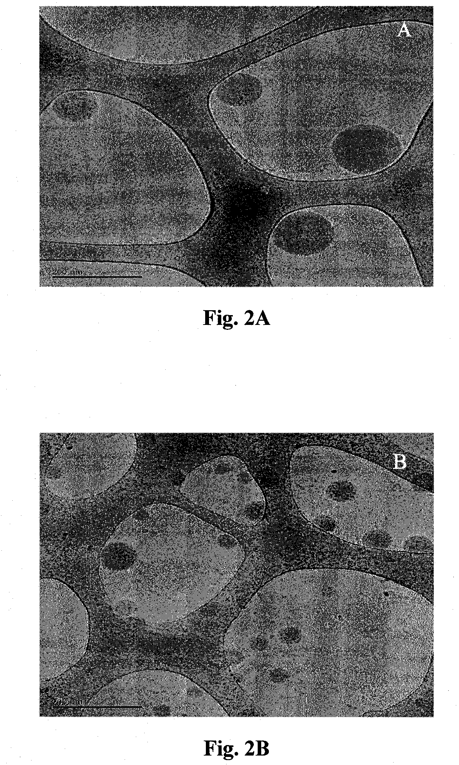

DHEA was encapsulated within the oil core of PLGA (4500 or 50000Da) nanocapules. The Cryo-TEM images at various areas of the carbon grid are depicted in **Figs. 2A** and **2B**. The nanocapsules appear spherical and nanometric and no DHEA crystals were observed.

For encapsulation efficiency and active substance content determination, [³H] DHEA was incorporated within MCT NCs. The initial theoretical DHEA content for the cationic and anionic NCs, following diafiltration with PBS (pH 7.4), were 0.49 and 0.52%, while the observed contents were 0.18 and 0.15% respectively. The encapsulation efficiency was therefore 36.5 and 30.4% for the positively and negatively charged NCs, respectively (as shown in **Table 5**).

**Table 5: DHEA content and loading efficiency within MCT NCs**

| Formulation | Theoretical conc. (%, w/v) | Observed conc. (%, w/v) | Yield (%) |
|---|---|---|---|
| *Positively charged [³H]DHEA loaded MCT NCs* | 0.013 | 0.006 | 36.53 |
| *Negatively charged [³H]DHEA loaded MCT NCs* | 0.013 | 0.005 | 30.40 |

### Skin penetration of fluorescent labeled nanospheres (reference example)

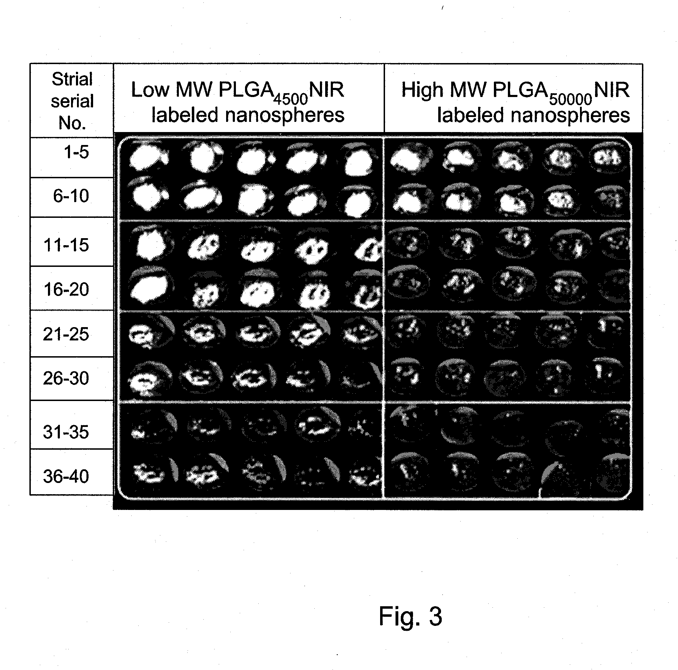

To evaluate skin penetration of NPs, nanospheres comprising of PLGA₄₅₀₀ or PLGA₅₀₀₀₀ were prepared, while a quantity of the polymer was covalently labeled with the infra-red dye NIR-783. Fluorescent formulations were topically administered on abdominal human skin of 60 years old male, using Franz cells (2.25mg/cm²). After 3h, skin specimens were washed and scanned using ODYSSEY® Infra Red Imaging System (LI-COR Biosciences, NE, USA). Fluorescent images of various consecutive tape stripping following topical administration are presented in **Fig. 3**. Without being bound to theory, the results suggest that PLGA₄₅₀₀ penetrate deeper than PLGA₅₀₀₀₀ into the skin layers. This may be attributed to the more rapid biodegradation of PLGA₄₅₀₀ compared to PLGA₅₀₀₀₀

### Skin penetration of fluorescent labeled nanocapsules (reference example)

To evaluate skin penetration of nanocapsules (NCs), as compared to nanospheres (NSs), formulations were incorporated with the fluorescent probe DiD. In order to define the bio-fate of PLGA nanocarrier, DiD fluorescent-probe-loaded-MCT NCs coated with PLGA covalently bound to rhodamine B were prepared. In the absence of MCT, NPs were formed. Non-degradable commercially available rhodamine B loaded Latex nanospheres were also investigated.

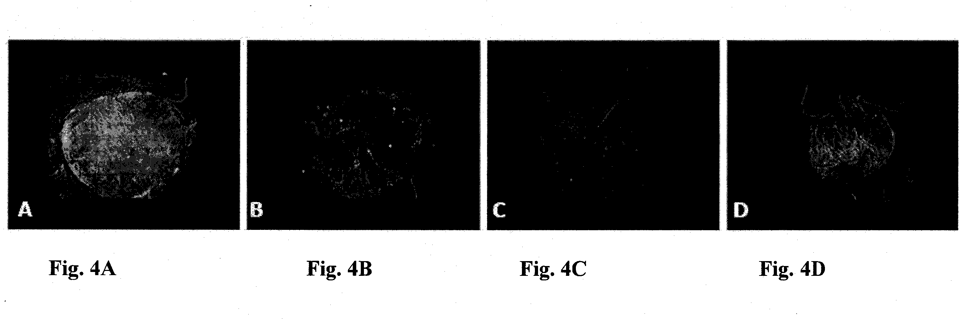

The fluorescent formulations were topically administered on abdominal human skin of 40 years old female, using Franz cells (4.5mg/cm²). After 2 h, skin specimens were washed and scanned using Zeiss LSM710 confocal laser scanning microscope. Reconstructed fluorescent images of whole skin specimens are depicted in **Figs. 4A-D**. The results clearly indicate that all DiD loaded nanoparticles elicited larger fluorescent values as compared to DiD control solution. In addition, PLGA₄₅₀₀ nanocapsules exhibited superior skin penetration/retention as compared to other nanoparticulate delivery systems.

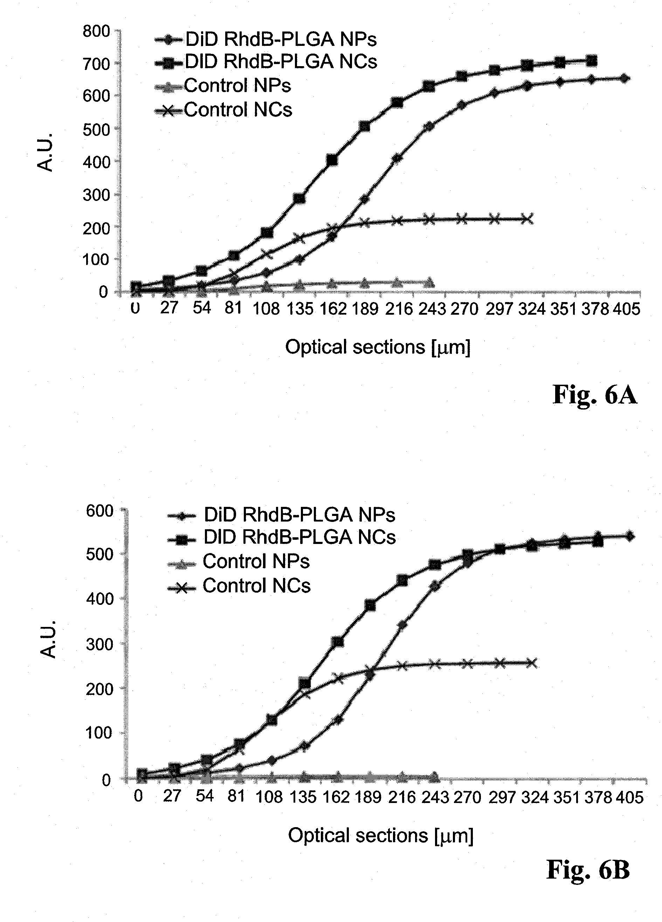

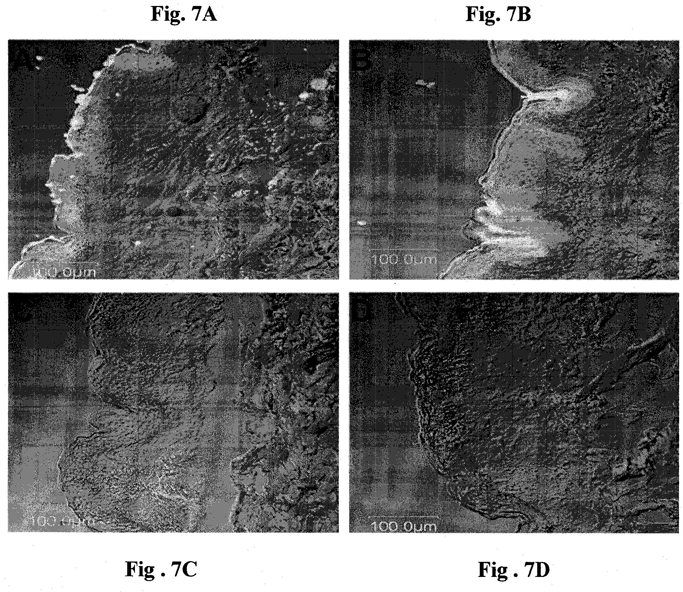

The dually labeled nanocarriers formulations and their respective controls were applied for 2 hours on abdominal human skin of 50 years old female. Reconstructed fluorescent images of whole skin specimens are depicted in **Figs. 5A-****E.** The 3D of the NPs and NCs following 2 hours of topical treatment showed that more of the fluorescent cargo was released from NCs than NPs although both reached the same depth (close to 200 µm), while the respective controls remained on the superficial skin layers. The results clearly indicate that DiD loaded nanoparticles penetrates at the same fashion as was previously described. Furthermore, rhodamine B intensity, which originally derived from the fluorescent probe conjugation to PLGA, was much higher when the PLGA based nanoparticulate carriers were topically administered as compared to their respective treatments (**Figs. 6A-B**), as was also depicted in the cross section images (**Figs. 7A-D**).

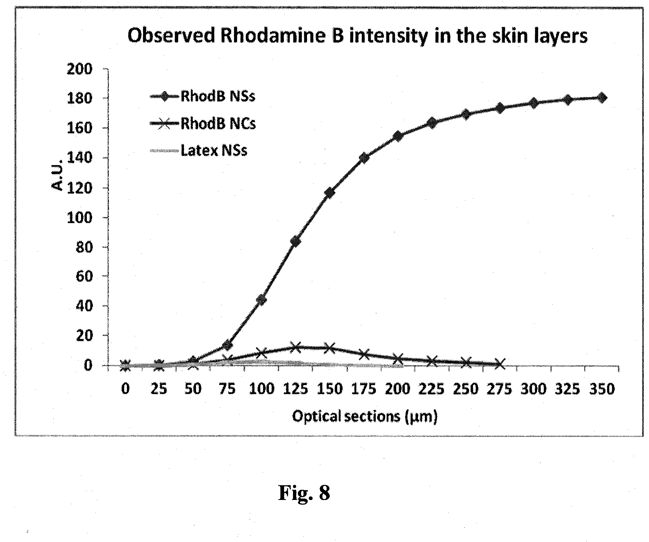

Finally, poor rhodamine B intensity was recorded following 2 hours incubation of non-degradable rhodamine B latex NSs on abdominal human skin of 30 years female. This result suggests that non-degradable based carrier has a major limit to release its cargo when compared to degradable systems (**Fig 8**).

### [³H]DHEA NCs site localization and deep skin layer localization (reference example)

The results reported in **Figs. 9A-D** show the *ex-vivo* dermato-biodistribution in the skin compartments of [³H]DHEA following topical application of negatively and positively charged [³H]DHEA loaded PLGA NCs and their respective controls at different incubation periods. Above 90% from the initial amount applied of the radiolabeled DHEA, from the different oil controls were recovered from the donor cell at each time interval up to 24h. When DHEA loaded NCs were applied, again, most of the radioactive compound was collected at the donor compartment, with an average of over 90% up to 6 hours, with a notable decrease to approximately 80, 65 and 55% recorded at 8, 12 and 24 hours, respectively. [³H]DHEA distribution in the upper skins layers as a function of SC depth following a sequential 10 tape stripping (TS) is depicted in **Table 6**. Each pair of TS was extracted and analyzed by liquid scintillation, resulting in a sequence of five sub-layers description of the SC from each specimen. Regardless to the treatment applied, it can be noted that the highest levels of [³H]DHEA were detected in layers A and B, which represents the outermost layers of the SC, with a coordinate decrease recorded at the inner layers C, D and E. Time related accumulation of the radioactive compound in the different SC layers occurred when the negatively and positively charged [³H]DHEA loaded NCs were applied. It should be noted that irrespective of the formulation, the concentration of radioactivity within the SC was low (around 1-2%). It can clearly be observed that at 24 h post application, the concentration of radioactivity diminished progressively in the internal layers (**Table 6**) of the SC. However, marked differences between the DHEA loaded NCs and their respective controls were recorded in the viable skin compartments (epidermis and dermis). [³H]DHEA levels reached a plateau of -3% and 5.5% in the epidermis and dermis respectively, following 6 hours incubation of both positively and negatively charged DHEA NCs (**Figs. 9**), while [³H]DHEA levels obtained in the epidermis and dermis with the respective oil controls did not reach 1% over all the treatment periods up to 24h (**Figs. 9**) (*P<0.05*).

**Table 6: [³H]DHEA distribution over time in the different SC layers of porcine skin following incubation with different nanocapsule formulations. Values are mean ± SD. N=4**

| **Formulation** | **Incubation periods (hours)** | **Stratum corneum layers (strips number)** | | | | |
|---|---|---|---|---|---|---|
| | | **A (1-2)** | **B (3-4)** | **C (5-6)** | **D (7-8)** | **E (9-10)** |
| *Positively charged [³H]DHEA loaded MCT NCs* | 1 | 0.2%±0.0 | 0.2%±0.1 | 0.1 %±0.0 | 0.1 %±0.1 | 0.1%±0.1 |
| | 3 | 0.3%±0.2 | 0.2%±0.1 | 0.1%±0.1 | 0.1%±0.1 | 0.1%±0.1 |
| | 6 | 0.3%±0.1 | 0.1%±0.1 | 0.1%±0.1 | 0.1%±0.1 | 0.1%±0.1 |
| | 8 | 0.7%±0.6 | 0.3%±0.2 | 0.2%±0.1 | 0.1%±0.1 | 0.1%±0.1 |
| | 12 | 2.0%±1.8 | 0.8%±0.7 | 0.3%±0.2 | 0.3%±0.2 | 0.2%±0.1 |
| | 24 | 1.9%±0.9 | 0.8%±0.1 | 0.6%±0.2 | 0.4%±0.1 | 0.3%±0.1 |
| *Negatively charged [³H]DHEA loaded MCT NCs* | 1 | 1.3%±0.1 | 0.4%±0.1 | 0.2%±0.1 | 0.1%±0.1 | 0.1%±0.0 |
| | 3 | 0.3%±0.0 | 0.2%±0.0 | 0.1%±0.0 | 0.1%±0.0 | 0.1%±0.0 |
| | 6 | 0.2%±0.1 | 0.2%±0.0 | 0.1%±0.0 | 0.1%±0.0 | 0.1%±0.0 |
| | 8 | 0.8%±0.8 | 0.3%±0.3 | 0.3%±0.1 | 0.2%±0.1 | 0.2%±0.1 |
| | 12 | 1.9%±1.3 | 0.8%±0.3 | 0.4%±0.1 | 0.3%±0.2 | 0.3%±0.2 |
| | 24 | 2.9%±1.8 | 1.4%±0.5 | 0.7%±0.3 | 0.5%±0.3 | 0.4%±0.2 |
| Positively charged oil control | 1 | 1.5%±0.9 | 1.1%±1.1 | 0.4%±0.5 | 0.2%±0.1 | 0.2%±0.1 |
| | 3 | 3.4%±1.4 | 1.4%±0.7 | 0.5%±0.3 | 0.2%±0.1 | 0.2%±0.1 |
| | 6 | 2.4%±0.8 | 0.8%±0.3 | 0.3%±0.1 | 0.3%±0.1 | 0.2%±0.1 |
| | 8 | 1.5%±0.5 | 0.7%±0.3 | 0.3%±0.1 | 0.2%±0.1 | 0.1%±0.1 |
| | 12 | 4.6%±1.7 | 1.4%±0.6 | 0.5%±0.2 | 0.3%±0.1 | 0.2%±0.1 |
| | 24 | 2.7%±0.8 | 0.9%±0.3 | 0.5%±0.2 | 0.3%±0.2 | 0.2%±0.2 |
| Negatively charged oil control | 1 | 2.2%±2.4 | 0.8%±0.7 | 0.2%±0.2 | 0.1%±0.0 | 0.1%±0.1 |
| | 3 | 1.7%±0.7 | 0.5%±0.2 | 0.2%±0.1 | 0.1%±0.1 | 0.1%±0.0 |
| | 6 | 1.1%±0.3 | 0.3%±0.0 | 0.1%±0.1 | 0.1%±0.1 | 0.1%±0.0 |
| | 8 | 1.3%±0.1 | 0.4%±0.1 | 0.2%±0.1 | 0.1%±0.1 | 0.1%±0.0 |
| | 12 | 1.0%±0.5 | 0.2%±0.1 | 0.1%±0.0 | 0.1%±0.0 | 0.0%±0.0 |
| | 24 | 2.0%±0.6 | 0.7%±0.2 | 0.3%±0.1 | 0.2%±0.1 | 0.1%±0.1 |

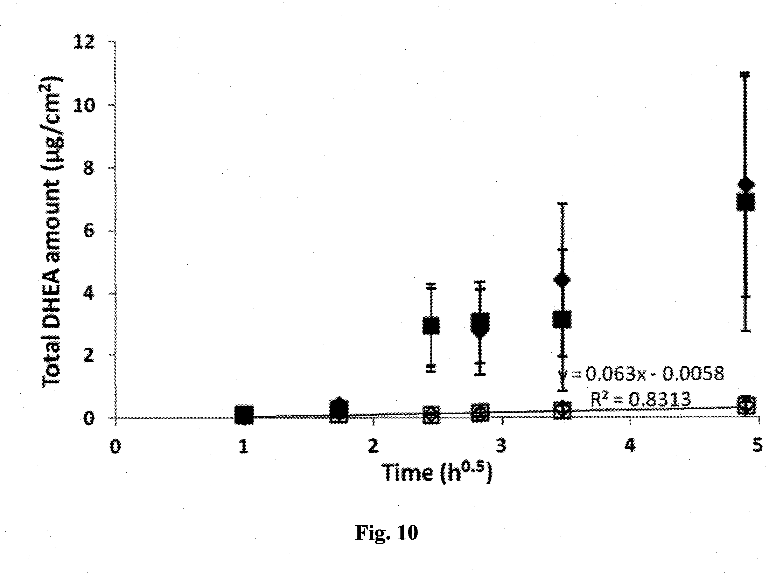

Increasing levels of the radioactive DHEA were found over time in the receptor compartment fluids when both positively and negatively DHEA loaded NCs were incubated, reaching 0.5%, 2.5% and 14% from the initial dose applied following 1 hour, 8 and 24 hours, respectively. On the other hand, the respective oil controls exhibited constant [³H]DHEA levels lower than 1% radioactivity at most time intervals. Although lag time of 3 hours was observed for the different formulations, [³H]DHEA appearance in the receptor fluids following positively and negatively NCs application was significantly higher than from the respective oil controls. The total amount of DHEA in the receptor fluids (µg/cm²), released from the different treatments, is plotted against the square root of time (**Fig. 10**). The low slow flux value 0.063 (µg/cm²/h^{0.5}), calculated from the slopes of the plotted graphs, for the oil controls correlates with their reported limited release profile. Then again, significant higher [³H]DHEA levels recorded in the receptor fluids when the negatively and positively DHEA NCs were topically applied, underlines a superior flux and superior percutaneous permeation of the drug when loaded into nanocarriers formulation. It should be emphasized that no significant difference between the two NCs formulation was observed at all time points indicating that the nature of the charge did not contribute to the enhanced skin penetration but rather the type of nanostructure used, i.e. vesicular nanocapsules.

The highly lipophilic radioactive compound, [³H]COE, was incorporated into PLGA NSs and MCT containing NCs, in an attempt to identify the fate of the empty nanocarrier when topically applied. Following diafiltration with PBS (pH=7.4) the encapsulation efficiency was 45% and 70% for the NSs and the NCs, respectively. Aqueous and oil controls of [³H]COE, without polymer, were prepared for the *ex-vivo* experiments. Again, over 90% from the initial amount of the tritiated COE were collected from the donor compartment following each incubation period, irrespective of the formulation type (data not shown). **Table 7** exhibits [³H]COE dermatobiodistribution as a function of the SC layers following the different treatments, as was previously described for [³H]DHEA. Up to 8 hours incubation of [³H]COE loaded NSs and NCs, less than 1% from the applied dose were extracted from the upper skin layers. Interestingly, a notable increase in layers A and B of was observed following 12 hours incubation of the NSs and NCs, similar to the previous observation reported when DHEA NCs were applied. Although no notable differences, associate to the incubation periods, in the levels of [³H]COE were recorded when the different controls were topically applied, the constant distribution of the [³H]COE in MCT was higher in comparison to the [³H]COE surfactant solution (**Table 7**). Finally, less than 0.5% of radioactivity was counted in the viable compartments (epidermis, dermis and receptor fluids) during the incubation periods, when both nanocarriers formulations and their respective control were applied (**Figs. 9**). It appears that more incubation time is needed to differentiate between the various formulations of COE.

**Table 7: [³H]COE distribution over time in the different SC layers of porcine skin following incubation with different nanocapsules formulations. Values are mean ± SD. N=3**

| **Formulation** | **Incubation periods (hours)** | **Stratum corneum layers (strips number)** | | | | |
|---|---|---|---|---|---|---|
| | | **A (1-2)** | **B (3-4)** | **C (5-6)** | **D (7-8)** | **E (9-10)** |
| *[³H]Cholesteryl oleyl ether loaded PLGA NSs* | 1 | 0.7%±0.8 | 0.2%±0.2 | 0.1%±0.1 | 0.1%±0.1 | 0.1%±0.1 |
| | 3 | 0.2%±0.1 | 0.2%±0.1 | 0.1%±0.1 | 0.1%±0.0 | 0.1%±0.0 |
| | 6 | 0.3%±0.2 | 0.2%±0.2 | 0.1%±0.1 | 0.1%±0.1 | 0.1%±0.1 |
| | 8 | 0.9%±1.0 | 0.3%±0.4 | 0.1%±0.1 | 0.1%±0.1 | 0.1%±0.1 |
| | 12 | 0.9%±1.3 | 0.4%±0.5 | 0.4%±0.5 | 0.2%±0.2 | 0.1%±0.2 |
| | 24 | 3.6%±0.7 | 1.5%±0.8 | 0.9%±0.5 | 0.7%±0.4 | 0.5%±0.4 |
| *[³H]Cholesteryl oleyl ether loaded PLGA NCs* | 1 | 0.2%±0.2 | 0.1%±0.0 | 0.1%±0.0 | 0.0%±0.0 | 0.0%±0.0 |
| | 3 | 0.4%±0.5 | 0.1%±0.1 | 0.1%±0.0 | 0.0%±0.0 | 0.0%±0.0 |
| | 6 | 0.4%±0.6 | 0.1%±0.1 | 0.2%±0.3 | 0.1%±0.1 | 0.0%±0.0 |
| | 8 | 0.6%±0.7 | 0.2%±0.2 | 0.1%±0.2 | 0.1%±0.1 | 0.1%±0.1 |
| | 12 | 1.2%±0.8 | 0.4%±0.4 | 0.2%±0.1 | 0.2%±0.2 | 0.1%±0.1 |
| | 24 | 2.4%±1.8 | 0.8%±0.6 | 0.4%±0.3 | 0.3%±0.2 | 0.2%±0.2 |
| *[³H]Cholesteryl oleyl ether surfactant solution* | 1 | 0.4%±0.8 | 0.2%±0.3 | 0.2%±0.3 | 0.1%±0.2 | 0.3%±0.6 |
| | 3 | 0.1%±0.1 | 0.1%±0.1 | 0.1%±0.0 | 0.0%±0.0 | 0.0%±0.0 |
| | 6 | 0.2%±0.1 | 0.1%±0.1 | 0.1%±0.0 | 0.1%±0.0 | 0.1%±0.0 |
| | 8 | 0.5%±0.5 | 0.3%±0.3 | 0.3%±0.3 | 0.1%±0.2 | 0.1%±0.1 |
| | 12 | 0.5%±0.4 | 0.3%±0.2 | 0.2%±0.2 | 0.1%±0.1 | 0.1%±0.1 |
| | 24 | 0.5%±0.1 | 0.3%±0.2 | 0.2%±0.1 | 0.2%±0.1 | 0.1%±0.1 |
| *[³H]Cholesteryl oleyl ether oil control* | 1 | 1.8%±0.5 | 0.6%±0.4 | 0.3%±0.2 | 0.1%±0.1 | 0.1%±0.0 |
| | 3 | 1.0%±0.7 | 0.4%±0.3 | 0.1%±0.1 | 0.1%±0.0 | 0.0%±0.0 |
| | 6 | 1.2%±0.3 | 0.4%±0.2 | 0.1%±0.0 | 0.1%±0.0 | 0.1%±0.0 |
| | 8 | 1.7%±0.5 | 0.8%±0.5 | 0.3%±0.1 | 0.1%±0.1 | 0.1%±0.0 |
| | 12 | 1.2%±0.5 | 0.7%±0.2 | 0.2%±0.1 | 0.1%±0.1 | 0.1%±0.1 |
| | 24 | 1.6%±0.3 | 0.5%±0.3 | 0.3%±0.1 | 0.1%±0.1 | 0.1%±0.0 |

### Thiol surface activated NPs and MAbs conjugated NPs (reference example)

Thiol surface activated NPs were prepared from the following polymers:
- PLGA of a MW of approximately 48,000 Da,
- PEG-PLGA_{50,000} and PLGA₄₅₀₀,
- PEG-PLA_{100,000}

### Preparation of immunoNPs conjugated to various MAbs (reference example)

The following MAbs were successfully conjugated to the surface of the thiolated NPs with high conjugation efficiency (see **Table 8**):
- Cetuximab
- Rituximab
- Herceptin
- Avastin

**Table 8: Properties of INPs conjugated to relevant MAbs**

| ***Polymer*** | ***MAb*** | ***Size (nm)*** | ***Zeta potential (mV)*** | ***Conjugation (%)*** |
|---|---|---|---|---|
| PEG-PLGA_{50,000} /PLGA_{48,000} | Cetuximab | 75 | -46 | 93 |
| PEG-PLGA_{50,000} /PLGA_{48,000} | Rituximab | 73.75 | N.A | 86.7% |

### Morphological evaluation using TEM (reference example)

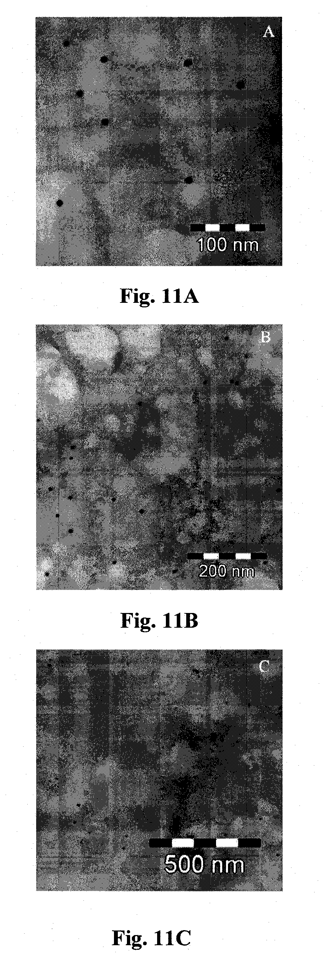

The coupling of cetuximab MAb to INPs was qualitatively confirmed by TEM observations, using 12 nm gold labeled goat anti-human IgG (Jackson ImmunoResearch Laboratories, PA, USA). Each gold black spot observed in **Figs. 11A-C** represents MAb molecule attached to the INPs surfaces sites that reacted with the gold labeled IgG. It can be deduced that the MAb was conjugated to the surface of the INPs by the linker and the reaction conditions did not affect the affinity of the MAb to the secondary antibody.

### Binding capacity determination in vitro in A549 cell line by Flow cytometry

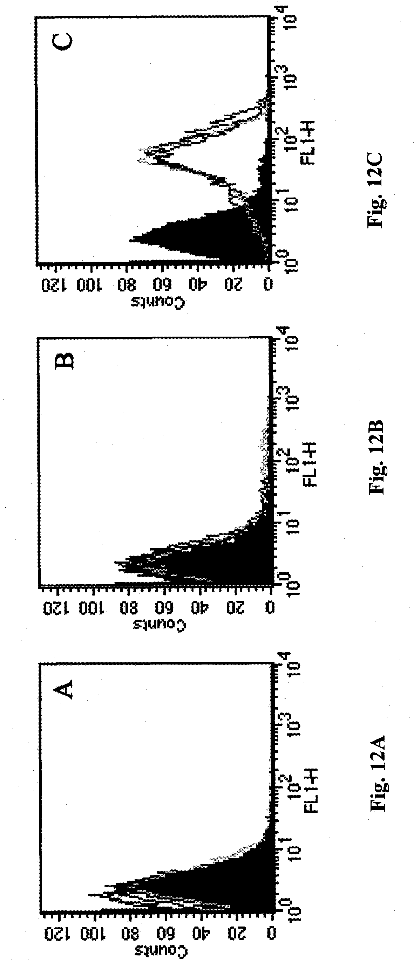

For evaluation of the binding properties evaluation using flow cytometry, cells were detached using a 0.05% solution of EDTA. Cells were re-suspended in FACS medium (1% BSA, 0.02% Sodium Azide in PBS). 200,000 cells in 200µl were used for each treatment. Cells were centrifuged at 1200 rpm at 4 degrees. Then, cells were incubated with either native cetuximab antibody or equivalent concentrations of cetuximab immunonanoparticles over ice, for 1h. 0.005 µg/ml, 0.01 µg/ml, 0.025 µg/ml, 0.05 µg/ml, 0.1 µg/ml and 0.5 µg/ml cetuximab antibody or INPs equivalents were used. The anti-CD-20 antibody, rituximab (Mabthera®) was used as an isotype matched irrelevant nonbinding control. Cells were also incubated with equivalent concentrations of surface activated NPs and rituximab INPs as negative controls, to exclude non specific binding of INPs. Following 1h incubation, cells were centrifuged and washed twice with FACS medium. Cells were then incubated for forty minutes at 4°C in the dark with FITC-conjugated AffiniPure F(ab)'₂ Fragment goat anti-human IgG (Jackson Immunoresearch). Cells were then centrifuged and washed twice with FACS medium. Cells were re-suspended in FACS medium and fluorescence was determined by flow cytometry. The results are depicted in **Figs. 12A-C**. The results clearly indicate that cetuximab immunonanoparticles exhibited excellent binding properties, at all MAb concentrations evaluated. Non specific binding was eliminated by cell incubation of both surface activated NPs (thiol bearing NPs) and isotype matched rituximab immunonanoparticles.

### Skin penetration of mmunoparticles (reference example)

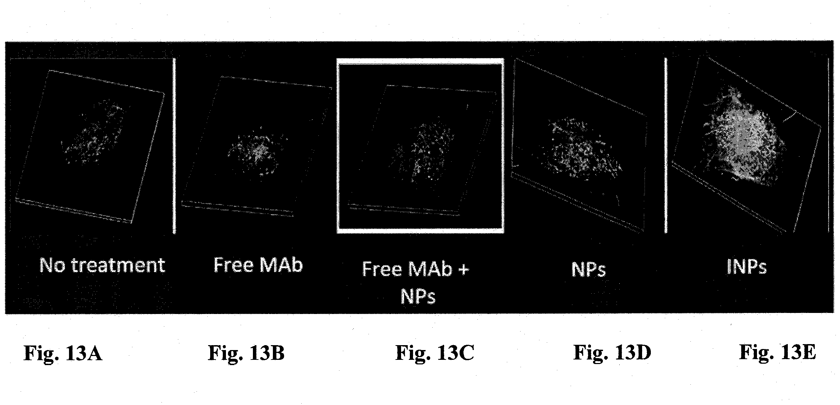

To evaluate the ability of nanoparticles to enhance the penetration of macromolecules into the skin, INPs covalently conjugated to cetuximab MAb were prepared. 6mg/cm² equivalent to 0.12 mg MAb/cm² were topically administered to 44 years old female abdominal human skin, over 3 h, against relevant controls. Then, skin specimens were washed and immunostained with Cy5 labeled goat anti-human secondary IgG (Jackson ImmunoResearch Laboratories, PA, USA). Reconstructed fluorescent images were performed using an Olympus confocal Microscope (**Figs. 13A-E**).

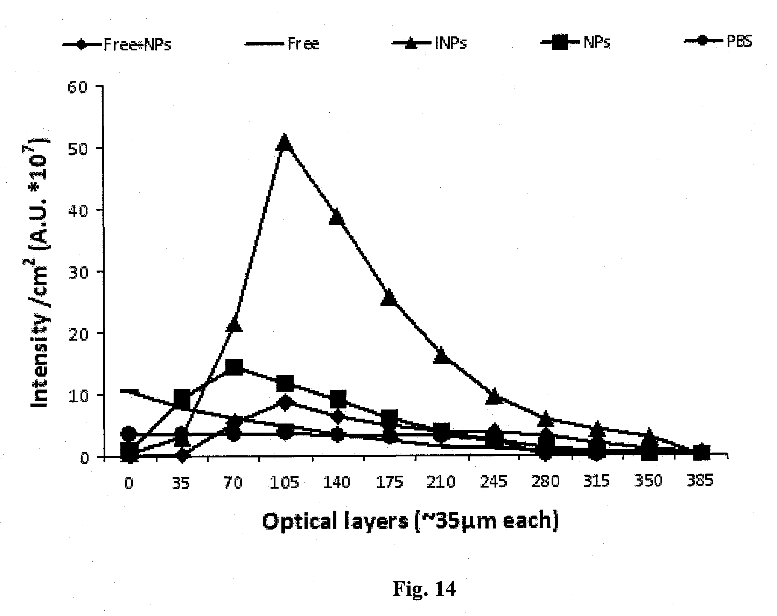

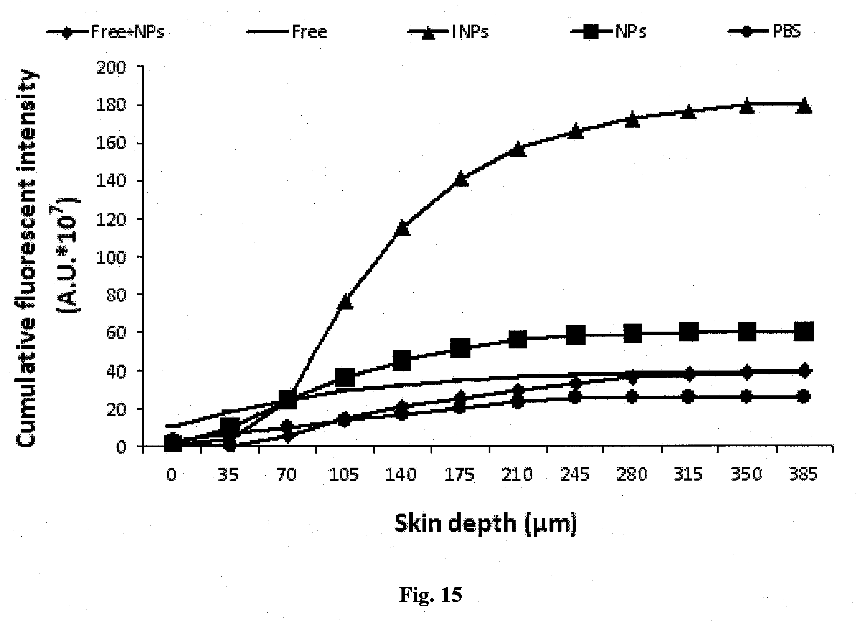

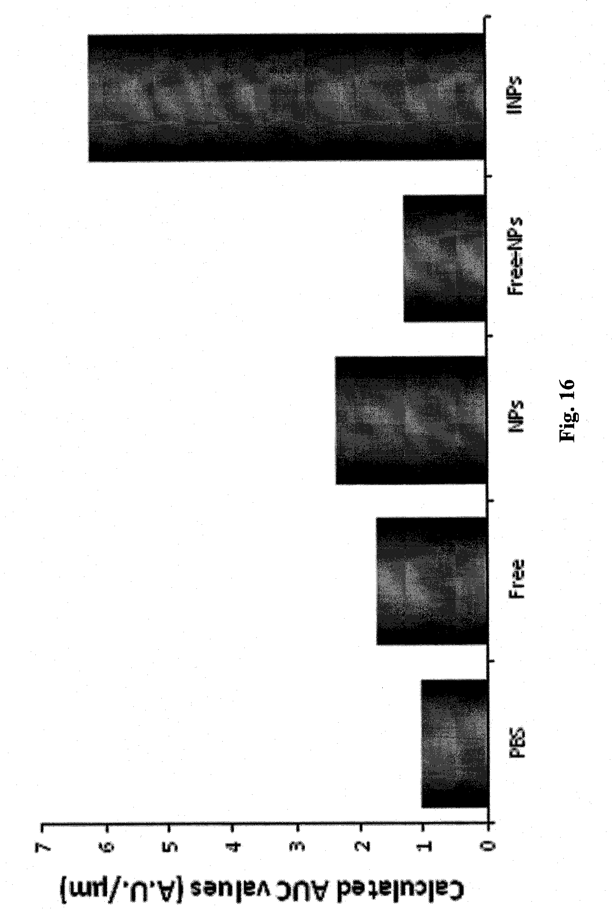

**Figs. 14** and **15** deal with the same experiment. It can be noted qualitatively and quantitatively) that the NPs and the INPs elicited the more intense fluorescent values with a more preannounced effect for the INPs as compared to NPs. **Fig. 14** clearly demonstrates the most marked quantitative fluorescent intensity per cm² elicited by the INPs. From **Fig. 15** and **Fig. 16** it can be observed that INPs elicited the highest cumulative intensity per cm², clearly indicating that the NPs promote MAb skin penetration/retention.

## Claims

1. A poly(lactic glycolic) acid (PLGA) nanoparticle having an average diameter of at most 500 nm, the PLGA having an average molecular weight of between 2,000 and 20,000 Da, wherein said nanoparticle being associated with at least one agent selected from
(i) a hydrophilic therapeutic agent being conjugated to or associated with the surface of said nanoparticle wherein the at least one hydrophilic therapeutic agent is associated with the nanoparticle via one or more linker moieties, wherein the linker is a fatty cystein having an alkyl chain of at least 10 carbon atoms, and
(ii) a lipophilic therapeutic agent being contained within said nanoparticle wherein said nanoparticle is further associated with at least one non-active agent, wherein the non-active agent is an alkyl amino acid, wherein the alkyl portion of said alkyl amino acid has between 10 and 30 carbon atoms and may be linear or branched, saturated, semi saturated or unsaturated.

2. The nanoparticle according to claim 1, wherein the PLGA has an average molecular weight of between 2,000 and 10,000 Da, or between 2,000 and 7,000 Da, or between 2,000 and 5,000 Da, or between 4,000 and 20,000 Da, or between 4,000 and 10,000 Da, or between 4,000 and 5,000 Da.

3. The nanoparticle according to claim 1, wherein the PLGA is a random copolymer of equimolar PLA and PGA, having a molecular weight of at least 4,500 Da, wherein the nanoparticle has an average diameter between 100 and 200 nm and is optionally in the form of a nanocapsule or a nanosphere.

4. The nanoparticle according to claim 1 or 3, wherein the at least one therapeutic agent is a macromolecule.

5. The nanoparticle according to claim 1, being in the form of a nanocapsule.

6. The nanoparticle according to claim 1, being in the form of a nanosphere.

7. The nanoparticle according to any one of claims 1 to 6, wherein the nanoparticle is PEGylated with polyethylene glycols (PEG) or non-PEGylated.

8. The nanoparticle according to claim 1, wherein the nanoparticle contains one hydrophobic agent in the nanoparticle's core, and one hydrophilic agent in the shell, extending out of the nanoparticle or associated with the linker.

9. The nanoparticle according to any one of claims 1 to 8, wherein the linker is oleylcysteineamide.

10. A composition comprising at least one nanoparticle according to any one of claims 1 to 9, said composition being optionally a pharmaceutical composition.

11. The composition according to claim 10, being adapted for transdermal administration of a therapeutic agent, for topical administration of a therapeutic agent across skin layers, for delivery of a therapeutic agent across the Stratum Corneum, for administration of a therapeutic agent by injection, for oral administration of a therapeutic agent, for ophthalmic administration of a therapeutic agent, or for ophthalmic administration via injection.

12. The composition according to claim 10 or 11, being essentially free of water.

13. A poly(lactic glycolic) acid (PLGA) nanoparticle, the PLGA having an average molecular weight of between 2,000 and 20,000 Da, said nanoparticle being surface-conjugated to at least one agent, said conjugation being via a linker moiety being a fatty cysteine having an alkyl chain of at least 10 carbon atoms, the nanoparticle having an average diameter of at most 500 nm, the nanoparticles being obtainable by a process comprising:
- obtaining a PLGA nanoparticle having an average diameter of at most 500 nm, the PLGA having an average molecular weight of between 2,000 and 20,000 Da;
- reacting said nanoparticle with the linker moiety under conditions permitting association between the nanoparticle surface and the linker moiety, to thereby obtain a surface-modified nanoparticle; and
- contacting the surface-modified nanoparticle with at least one agent being selected from a therapeutic or non-active agent, to allow association between the linker end group with said at least one agent.

14. A process for the preparation of a poly(lactic glycolic) acid (PLGA) nanoparticle, the PLGA having an average molecular weight of between 2,000 and 20,000 Da, said nanoparticle being surface-associated to at least one agent, said surface-association being via a linker moiety being a cysteine having an alkyl chain of at least 10 carbon atoms, the nanoparticle having an average diameter of at most 500 nm, the process comprising:
- obtaining a PLGA nanoparticle having an average diameter of at most 500 nm, the PLGA having an average molecular weight of between 2,000 and 20,000 Da;
- reacting said nanoparticle with the linker moiety under conditions permitting association between the nanoparticle surface and the linker moiety, to thereby obtain a surface-modified nanoparticle; and
- contacting the surface-modified nanoparticle with at least one agent being selected from a therapeutic or non-active agent, to allow association between the linker end group with said at least one agent.

## Patentansprüche

1. Poly(lactid-co-Glycolid) (PLGA)-Nanopartikel mit einem mittleren Durchmesser von höchstens 500 nm, wobei das PLGA ein mittleres Molekulargewicht zwischen 2.000 und 20.000 Da hat, wobei das Nanopartikel mit mindestens einem Agens assoziiert ist, das ausgewählt ist aus
(i) einem hydrophilen therapeutischen Agens, das an die Oberfläche des Nanopartikels konjugiert ist oder mit dieser assoziiert ist, wobei das mindestens eine hydrophile therapeutische Agens mit dem Nanopartikel über eine oder mehrere Linker-Gruppen assoziiert ist, wobei der Linker ein fettes Cystein mit einer Alkylkette von mindestens 10 Kohlenstoffatomen ist, und
(ii) einem lipophilen therapeutischen Agens, das in dem Nanopartikel enthalten ist, wobei das Nanopartikel des Weiteren mit mindestens einem nicht-aktiven Agens assoziiert ist, wobei das nicht-aktive Agens eine Alkylaminosäure ist, wobei der Alkylteil der Alkylaminosäure zwischen 10 und 30 Kohlenstoffatome aufweist und linear, verzweigt, gesättigt, halbgesättigt oder ungesättigt sein kann.

2. Nanopartikel nach Anspruch 1, wobei das PLGA ein mittleres Molekulargewicht zwischen 2.000 und 10.000 Da oder zwischen 2.000 und 7.000 Da oder zwischen 2.000 und 5.000 Da oder zwischen 4.000 und 20.000 Da oder zwischen 4.000 und 10.000 Da oder zwischen 4.000 und 5.000 Da hat.

3. Nanopartikel nach Anspruch 1, wobei das PLGA ein Random-Copolymer aus equimolarem PLA und PGA mit einem Molekulargewicht von mindestens 4.500 Da, wobei das Nanopartikel einen mittleren Durchmesser zwischen 100 und 200 nm hat und gegebenenfalls in Form einer Nanokapsel oder einer Nanosphäre vorliegt.

4. Nanopartikel nach Anspruch 1 oder 3, wobei das mindestens eine therapeutische Agens ein Makromolekül ist.

5. Nanopartikel nach Anspruch 1, das in der Form einer Nanokapsel vorliegt.

6. Nanopartikel nach Anspruch 1, das in der Form einer Nanosphäre vorliegt.

7. Nanopartikel nach einem der Ansprüche 1 bis 6, wobei das Nanopartikel mit Polyethylenglycolen (PEG) PEGyliert ist oder nicht PEGyliert ist.

8. Nanopartikel nach Anspruch 1, wobei das Nanopartikel ein hydrophobes Agens im Kern des Nanopartikels enthält und ein hydrophiles Agens in der Hülle enthält, welches sich aus dem Nanopartikel heraus erstreckt oder mit dem Linker assoziiert ist.

9. Nanopartikel nach einem der Ansprüche 1 bis 8, wobei der Linker Oleylcysteinamid ist.

10. Zusammensetzung, die mindestens ein Nanopartikel nach einem der Ansprüche 1 bis 9 umfasst, wobei die Zusammensetzung gegebenenfalls ein Arzneimittel ist.

11. Zusammensetzung nach Anspruch 10, die für die transdermale Verabreichung eines therapeutischen Agens, für die topische Verabreichung eines therapeutischen Agens durch Hautschichten, für die Abgabe eines therapeutischen Agens durch das Stratum Corneum, für die Verabreichung eines therapeutischen Agens durch Injektion, für die orale Verabreichung eines therapeutischen Agens, für die ophthalmische Verabreichung eines therapeutischen Agens oder für die ophthalmische Verabreichung mittels Injektion angepasst ist.

12. Zusammensetzung nach Anspruch 10 oder 11, die im Wesentlichen frei von Wasser ist.

13. Poly(lactid-co-Glycolid) (PLGA)-Nanopartikel, wobei das PLGA ein mittleres Molekulargewicht zwischen 2.000 und 20.000 Da hat, wobei das Nanopartikel an mindestens ein Agens oberflächenkonjugiert ist, wobei die Konjugation über eine Linkergruppe erfolgt, die ein fettes Cystein ist, das eine Alkylkette mit mindestens 10 Kohlenstoffatomen aufweist, wobei das Nanopartikel einen mittleren Durchmesser von höchstens 500 nm hat, wobei die Nanopartikel mithilfe eines Verfahrens erhältlich sind, das umfasst:
- Erhalten eines PLGA-Nanopartikels, das einen mittleren Durchmesser von höchstens 500 nm hat, wobei das PLGA ein mittleres Molekulargewicht zwischen 2.000 und 20.000 Da hat;
- Umsetzen des Nanopartikels mit der Linkergruppe unter Bedingungen, die die Assoziation zwischen der Nanopartikeloberfläche und der Linkergruppe ermöglichen, um dadurch ein oberflächenmodifiziertes Nanopartikel zu erhalten; und
- Inkontaktbringen des oberflächenmodifizierten Nanopartikels mit mindestens einem Agens, das aus einem therapeutischen oder nicht-aktiven Agens ausgewählt ist, um die Assoziation zwischen der Linkerendgruppe mit dem mindestens einen Agens zu ermöglichen.

14. Verfahren zur Herstellung eines Poly(lactid-co-Glycolid) (PLGA)-Nanopartikels, wobei das PLGA ein mittleres Molekulargewicht zwischen 2.000 und 20.000 Da hat, wobei das Nanopartikel mit mindestens einem Agens oberflächenassoziiert ist, wobei die Oberflächenassoziation über eine Linkergruppe erfolgt, die ein Cystein mit einer Alkylkette mit mindestens 10 Kohlenstoffatomen ist, wobei das Nanopartikel einen mittleren Durchmesser von höchstens 500 nm hat, wobei das Verfahren umfasst:
- Erhalten eines PLGA-Nanopartikels, das einen mittleren Durchmesser von höchstens 500 nm hat, wobei das PLGA ein mittleres Molekulargewicht zwischen 2.000 und 20.000 Da hat;
- Umsetzen des Nanopartikels mit der Linkergruppe unter Bedingungen, die die Assoziation zwischen der Nanopartikeloberfläche und der Linkergruppe ermöglichen, um dadurch ein oberflächenmodifiziertes Nanopartikel zu erhalten; und
- Inkontaktbringen des oberflächenmodifizierten Nanopartikels mit mindestens einem Agens, das aus einem therapeutischen oder nicht-aktiven Agens ausgewählt ist, um die Assoziation zwischen der Linkerendgruppe mit dem mindestens einen Agens zu ermöglichen.

## Revendications

1. Nanoparticule d'acide polylactique glycolique (PLGA) ayant un diamètre moyen d'au maximum 500 nm, le PLGA ayant un poids moléculaire moyen compris entre 2000 et 20000 Da, ladite nanoparticule étant associée à au moins un agent choisi parmi
(i) un agent thérapeutique hydrophile conjugué ou associé à la surface de ladite nanoparticule, le au moins un agent thérapeutique hydrophile étant associé à la nanoparticule par le biais d'une ou de plusieurs fractions de liaison, le lieur étant une cystéine grasse ayant une chaîne alkyle d'au moins 10 atomes de carbone, et
(ii) un agent thérapeutique lipophile contenu à l'intérieur de ladite nanoparticule, ladite nanoparticule étant en outre associée à au moins un agent non actif, l'agent non actif étant un acide aminé alkyle, la portion alkyle dudit acide aminé alkyle comportant entre 10 et 30 atomes de carbone et pouvant être linéaire, ramifiée, saturée, semi-saturée ou insaturée.

2. Nanoparticule selon la revendication 1, dans laquelle le PLGA a un poids moléculaire moyen compris entre 2 000 et 10 000 Da, ou entre 2 000 et 7 000 Da, ou entre 2 000 et 5 000 Da, ou entre 4 000 et 20 000 Da, ou entre 4 000 et 10 000 Da, ou entre 4 000 et 5 000 Da.

3. Nanoparticule selon la revendication 1, dans laquelle le PLGA est un copolymère aléatoire de PLA et PGA en quantité équimolaire, ayant un poids moléculaire d'au moins 4 500 Da, la nanoparticule ayant un diamètre moyen compris entre 100 et 200 nm et se présentant le cas échéant sous la forme d'une nanocapsule ou d'une nanosphère.

4. Nanoparticule selon la revendication 1 ou 3, dans laquelle le au moins un agent thérapeutique est une macromolécule.

5. Nanoparticule selon la revendication 1, se présentant sous la forme d'une nanocapsule.

6. Nanoparticule selon la revendication 1, se présentant sous la forme d'une nanosphère.

7. Nanoparticule selon l'une quelconque des revendications 1 à 6, la nanoparticule étant PEGylée avec des polyéthylène glycols (PEG) ou non PEGylée.

8. Nanoparticule selon la revendication 1, la nanoparticule contenant un agent hydrophobe dans le coeur de la nanoparticule, et un agent hydrophile dans l'enveloppe, s'étendant hors de la nanoparticule ou étant associée avec le lieur.

9. Nanoparticule selon l'une quelconque des revendications 1 à 8, dans laquelle le lieur est l'oléylcystéinamide.

10. Composition comprenant au moins une nanoparticule selon l'une quelconque des revendications 1 à 9, ladite composition étant le cas échéant une composition pharmaceutique.

11. Composition selon la revendication 10, adaptée pour l'administration transdermique d'un agent thérapeutique, pour l'administration topique d'un agent thérapeutique à travers les couches de la peau, pour la distribution d'un agent thérapeutique à travers la couche cornée, pour l'administration d'un agent thérapeutique par injection, pour l'administration par voie orale d'un agent thérapeutique, pour l'administration ophtalmique d'un agent thérapeutique, ou pour l'administration ophtalmique par injection.

12. Composition selon la revendication 10 ou 11, sensiblement dépourvue d'eau.

13. Nanoparticule d'acide polylactique glycolique (PLGA), le PLGA ayant un poids moléculaire moyen compris entre 2 000 et 20 000 Da, ladite nanoparticule étant conjuguée en surface à au moins un agent, ladite conjugaison étant par le biais d'une fraction de liaison qui est une cystéine grasse ayant une chaîne alkyle d'au moins 10 atomes de carbone, la nanoparticule ayant un diamètre moyen d'au maximum 500 nm, les nanoparticules pouvant être obtenues par un procédé comprenant :
- l'obtention d'une nanoparticule de PLGA ayant un diamètre moyen d'au maximum 500 nm, le PLGA ayant un poids moléculaire moyen compris entre 2 000 et 20 000 Da ;
- la réaction de ladite nanoparticule avec la fraction de liaison dans des conditions permettant l'association entre la surface de la nanoparticule et la fraction de liaison, ce qui permet ainsi d'obtenir une nanoparticule modifiée en surface ; et
- la mise en contact de la nanoparticule modifiée en surface avec au moins un agent qui est choisi parmi un agent thérapeutique ou un agent non actif, pour permettre l'association entre le groupe final de liaison et ledit au moins un agent.

14. Procédé pour la préparation d'une nanoparticule d'acide polylactique glycolique (PLGA), le PLGA ayant un poids moléculaire moyen compris entre 2 000 et 20 000 Da, ladite nanoparticule étant associée en surface à au moins un agent, ladite association de surface étant par le biais d'une fraction de liaison qui est une cystéine ayant une chaîne alkyle d'au moins 10atomes de carbone, la nanoparticule ayant un diamètre moyen d'au maximum 500 nm, le procédé comprenant :
- l'obtention d'une nanoparticule de PLGA ayant un diamètre moyen d'au maximum 500 nm, le PLGA ayant un poids moléculaire moyen compris entre 2 000 et 20 000 Da ;
- la réaction de ladite nanoparticule avec la fraction de liaison dans des conditions permettant l'association entre la surface de la nanoparticule et la fraction de liaison, ce qui permet ainsi d'obtenir une nanoparticule modifiée en surface ; et
- la mise en contact de la nanoparticule modifiée en surface avec au moins un agent qui est choisi parmi un agent thérapeutique ou un agent non actif, pour permettre l'association entre le groupe final de liaison et ledit au moins un agent.
